# EUROPEAN PATENT APPLICATION

(11) **EP 4 501 312 A1**
(43) Date of publication of application: **05.02.2025**
(21) Application number: 22933783.7
(22) Date of filing: 01.12.2022
(51) Int. Cl.: A61K 9/08, A61K 47/02, A61K 47/26, A61K 31/7088

(54) **LIQUID FORMULATION PHARMACEUTICAL COMPOSITION COMPRISING PLASMID DNA**

(30) Priority: 24.03.2022 KR 20220036970
(71) Applicant: Helixmith Co., Ltd, Seoul 07794 (KR)
(72) Inventor: BAE, Kyungdong, Yongin-si Gyeonggi-do 16978 (KR); JO, Yunyeol, Seongnam-si Gyeonggi-do 13178 (KR); PARK, Jihwan, Seoul 02591 (KR)
(74) Representative: J A Kemp LLP
(86) International application number: PCT/KR2022/019410
(87) International publication number: WO 2023/182614

(57) **Abstract**

The present invention relates to a liquid formulation pharmaceutical composition comprising plasmid DNA. The liquid formulation comprising plasmid DNA according to the present invention shows excellent efficacy in terms of storage stability of plasmid DNA, which is a pharmaceutically active ingredient, and accordingly, can be useful as a pharmaceutical composition with excellent product stability and safety.

## Description

### Technical Field

This patent application claims the benefit of and priority to Korean Patent Application No. 10-2022-0036970, filed on March 24, 2022, with the Korean Intellectual Property Office, the disclosure of which is incorporated herein by reference.

The present disclosure relates to a pharmaceutical composition in liquid formulation containing a plasmid DNA.

### Background Art

There is substantial clinical evidence that gene therapy involving the direct in vivo delivery of nucleic acid constructs that are not packaged in viruses or virus-like particles (commonly referred to as "naked" DNA or RNA constructs) can be effective in treating various diseases. For example, direct intramuscular injection of a DNA plasmid construct expressing two isoforms of human HGF protein (i.e., pCK-HGF-X7, also referred to as "VM202") has been shown to be effective in treating neuropathic pain. In a Phase II clinical trial, it was found that injecting VM202 into the calf muscles of patients with diabetic peripheral neuropathy significantly reduced pain, providing symptom relief sufficient to improve the quality of life for three months after a two-day treatment administered at two-week intervals. (Kessler et al., Annals Clin. Transl. Neurology 2(5):465-478 (2015)). The same DNA plasmid construct has also been found to be effective in treating patients with amyotrophic lateral sclerosis (ALS). In a Phase I clinical trial, nearly half of the ALS patients maintained a stable or improved condition after VM202 administration, with 47%, 50%, and 24% of subjects at 1, 2, and 3 months, respectively, experiencing no decline or an improvement in their Amyotrophic Lateral Sclerosis Functional Rating Scale-revised (ALSFRS-R) scores, which reflect the physical function of ALS patients, a better outcome than what was observed in historical controls (Robert L. Sufit et al., Amyotrophic Lateral Sclerosis and Frontotemporal Degeneration 18:269-278 (2017)).

However, the physicochemical stability of these DNA or RNA construct materials is known to be affected by the final preparation, formulation, and dosage form, as well as storage conditions. Typically, pharmaceutical formulations are provided in a lyophilized form for storage stability, but they must be reconstituted with injection water before administration to the patient. If the correct manual is not followed during reconstitution, the drug may not be uniformly reconstituted, leading to potential drug loss or contamination during the administration process. Therefore, if the drug can be provided in a liquid formulation rather than a lyophilized form, the safety and commercial competitiveness of the product can be enhanced. Thus, there is a need to develop liquid formulations of naked-DNA-based drugs with superior stability and safety.

### Disclosure of Invention

### Technical Problem

The present disclosure aims to provide a liquid formulation containing plasmid DNA.

### Solution to Problem

An aspect of the present disclosure provides a liquid formulation containing plasmid DNA.

As used herein, the term "liquid formulation" refers to a pharmaceutical composition that includes plasmid DNA and at least one pharmaceutically acceptable carrier or excipient and is in liquid form, as described herein.

The liquid formulation of the present disclosure relates to a universal liquid formulation of plasmid DNA that can be used regardless of the type of plasmid DNA. Thus, the liquid formulation of the present disclosure is not limited to the plasmid DNA (e.g., VM202) used in an embodiment of the present disclosure.

In an embodiment of the present disclosure, the plasmid DNA encodes human HGF or a variant thereof.

In an embodiment of the present disclosure, the plasmid DNA may be VM202, but is not limited thereto.

In an embodiment of the present disclosure, the plasmid DNA further includes a promoter. In the plasmid DNA, the promoter is operatively linked to a nucleic acid molecule encoding a protein. As used herein, the term "operatively linked" refers to a functional connection between a nucleic acid expression regulatory sequence (e.g., a promoter, signal sequence, or an array of transcription factor binding sites) and another nucleic acid sequence, whereby the regulatory sequence regulates the transcription and/or translation of the other nucleic acid sequence.

For example, if the vector of the present disclosure is an expression vector and a eukaryotic cell is used as a host, promoters derived from the genome of mammalian cells (e.g., metallothionein promoter, beta-actin promoter, human hemoglobin promoter, and human muscle creatine promoter) or promoters derived from mammalian viruses (e.g., adenovirus late promoter, vaccinia virus 7.5K promoter, SV40 promoter, cytomegalovirus promoter, HSV tk promoter, mouse mammary tumor virus (MMTV) promoter, HIV LTR promoter, Moloney virus promoter, Epstein-Barr virus (EBV) promoter, and Rous sarcoma virus (RSV) promoter) may be used, with a polyadenylation sequence generally used as a transcription termination sequence therein.

The expression vector of the present disclosure may include a selectable marker gene and/or a reporter gene as a selectable marker to evaluate whether the expression vector has been transformed and whether protein expression occurs.

The selectable marker genes include antibiotic resistance genes commonly used in the art, for example, genes conferring resistance to ampicillin, gentamicin, carbenicillin, chloramphenicol, streptomycin, kanamycin, geneticin, neomycin, and tetracycline. The reporter genes may include genes such as luciferase, beta-galactosidase, chloramphenicol acetyltransferase, or green fluorescent protein, but are not limited thereto.

Methods for introducing and expressing the recombinant vector of the present disclosure into cells are well known in the related technical field. The vector can be easily introduced into host cells, such as mammalian, bacterial, yeast, or insect cells, by methods known in the art. For example, the vector can be delivered into host cells by physical, chemical, or biological means. The physical means include calcium phosphate precipitation, lipofection, particle bombardment, microinjection, and electroporation. The chemical means include colloidal dispersion systems, such as macromolecular complexes, nanocapsules, microspheres, beads, water-in-oil emulsions, micelles, mixed micelles, and lipid-based systems, including liposomes. Furthermore, the biological means include the use of DNA or RNA vectors, such as the aforementioned lentivirus, retrovirus, etc., but are not limited thereto.

The term "VM202", as used herein, refers to a plasmid DNA, also called pCK-HGF-X7, which includes the pCK vector (SEQ ID NO: 6) and HGF-X7 (SEQ ID NO: 13) cloned into the pCK vector. VM202 was deposited on March 12, 2002, under accession number KCCM-10361 at the Korean Culture Center of Microorganisms (KCCM) in accordance with the Budapest Treaty.

The term "isoforms of hepatocyte growth factor" (HGF), as used herein, refers to polypeptides having an amino acid sequence that is at least 80% identical to the amino acid sequence of naturally occurring HGF polypeptides in animals. The term includes polypeptides having an amino acid sequence that is at least 80% identical to the full-length wild-type HGF polypeptide and polypeptides having an amino acid sequence that is at least 80% identical to a naturally occurring HGF allelic variant, splice variant, or deletion variant. In the present disclosure, the isoforms of HGF include at least two isoforms selected from the group consisting of full-length HGF (flHGF), deleted variant HGF (dHGF), NK1, NK2, and NK4. According to a more specific embodiment of the present disclosure, the isoforms of HGF used in the methods described herein include flHGF (SEQ ID NO: 1) and dHGF (SEQ ID NO: 2).

The terms "human flHGF," "flHGF," and "fHGF" are used interchangeably herein to refer to a protein consisting of amino acids 1-728 of the human HGF protein. The amino acid sequence of flHGF is provided as SEQ ID NO: 1.

The terms "human dHGF" and "dHGF" are used interchangeably herein to refer to a deleted variant of the HGF protein generated by alternative splicing of the human HGF gene. Specifically, "human dHGF" or "dHGF" refers to a human HGF protein with a deletion of five amino acids (F, L, P, S, and S) in the first kringle domain of the alpha chain from the full-length HGF sequence. Human dHGF is 723 amino acids long. The amino acid sequence of human dHGF is provided as SEQ ID NO: 2.

In an embodiment of the present disclosure, the plasmid DNA encodes SDF-1, IGF-1, c-Met, or fragments/isoforms/variants thereof.

The term "SDF-1" (stromal cell-derived factor 1), as used herein, is also known as C-X-C motif chemokine 12 (CXCL12) and refers to a chemokine protein encoded by the CXCL12 gene. SDF-1 is expressed in many cells and tissues and is distinguished as SDF-1 alpha and SDF-1 beta. SDF-1 is known to play a crucial role in angiogenesis, wound healing, neural development, and other processes.

An exemplary amino acid sequence of human SDF-1 alpha is provided as SEQ ID NO: 21.

An exemplary nucleotide sequence encoding the human SDF-1 alpha is provided as SEQ ID NO: 17.

The term "IGF-1" (insulin-like growth factor 1), as used herein, is also known as somatomedin C and refers to a hormone with a molecular structure similar to insulin. IGF-1 plays a significant role in promoting cell proliferation, particularly during childhood growth and development. IGF-1 is encoded by the IGF1 gene and is known to stimulate the growth of various cell types, inhibit apoptosis, and increase protein production within cells.

An exemplary amino acid sequence of the human IGF-1 Ea isoform is provided as SEQ ID NO: 19. An exemplary amino acid sequence of the human IGF-1 Ec isoform is provided as SEQ ID NO: 20.

The term "c-Met protein", as used herein, refers to tyrosine-protein kinase Met or hepatocyte growth factor receptor (HGFR). c-Met is encoded by the MET gene and is a kinase receptor with tyrosine kinase activity that is essential for embryonic development, tissue formation, cell division, and wound healing. In an embodiment of the present disclosure, the plasmid DNA may be pCK-SDF-1a (WO2016/048105A1), pTx-IGF-1X10 (WO2020/016655A2), pCMV3-cMet-flag (Sino Biological Inc., China), or a combination thereof, but is not limited thereto.

In an embodiment of the present disclosure, the pTx is a plasmid vector derived from pCK. pTx is generated by two sequential rounds of mutagenesis in pCK. It was created by removing unnecessary sequences between the kanamycin resistance gene and the ColE1 of pCK and by optimizing the length of the HCMV intron sequence.

In an embodiment of the present disclosure, the pCMV3 is a vector for mammalian protein expression driven by the CMV promoter.

In an embodiment of the present disclosure, the liquid formulation includes a plasmid DNA as an active ingredient of the pharmaceutical composition. The plasmid DNA may contain genetic material for gene therapy. Specifically, the plasmid DNA may encode a genetic material capable of correcting the function of defective genes or transcripts, or it may encode polypeptides, sense or antisense oligonucleotides, or RNA (coding or non-coding; for example, siRNA, shRNA, micro-RNA, and their antisense counterparts, such as antagoMiR). Formulations containing any plasmid DNA known in the art for use in gene therapy fall within the scope of the present disclosure.

In an embodiment of the present disclosure, the liquid formulation contains plasmid DNA at a concentration of 0.01-5 mg/mL. More specifically, it may include plasmid DNA at a concentration of 0.01 to 5 mg/mL, 0.01 to 3 mg/mL, 0.01 to 2 mg/mL, 0.01 to 1 mg/mL, 0.01 to 0.75 mg/mL, 0.01 to 0.6 mg/mL, 0.01 to 0.5 mg/mL, 0.1 to 5 mg/mL, 0.1 to 3 mg/mL, 0.1 to 2 mg/mL, 0.1 to 1 mg/mL, 0.1 to 0.75 mg/mL, 0.1 to 0.6 mg/mL, 0.1 to 0.5 mg/mL, 0.2 to 5 mg/mL, 0.2 to 3 mg/mL, 0.2 to 2 mg/mL, 0.2 to 1 mg/mL, 0.2 to 0.75 mg/mL, 0.2 to 0.6 mg/mL, 0.2 to 0.5 mg/mL, 0.3 to 5 mg/mL, 0.3 to 3 mg/mL, 0.3 to 2 mg/mL, 0.3 to 1 mg/mL, 0.3 to 0.75 mg/mL, 0.3 to 0.6 mg/mL, 0.3 to 0.5 mg/mL, or 0.5 mg/mL.

The plasmid DNA may be a polynucleotide of 2,000 to 15,000 base pairs, 2,000 to 10,000 base pairs, 2,000 to 9,000 base pairs, 2,000 to 8,000 base pairs, 2,000 to 7,000 base pairs, 2,000 to 6,000 base pairs, 2,000 to 5,000 base pairs, 3,000 to 15,000 base pairs, 3,000 to 10,000 base pairs, 3,000 to 9,000 base pairs, 3,000 to 8,000 base pairs, 3,000 to 7,000 base pairs, 3,000 to 6,000 base pairs, 3,000 to 5,000 base pairs, 4,000 to 8,000 base pairs, 4,000 to 7,500 base pairs, 4,000 to 6,000 base pairs, 6,000 to 9,000 base pairs, or 7,000 to 8,000 base pairs. The plasmid DNA may be a polynucleotide of a length that falls within the ranges provided herein.

The liquid formulation according to the present disclosure is designed to enhance the stability of the plasmid DNA. The stability of the plasmid DNA can be determined based on methods known in the art. In particular, stability can be determined based on the conformation of the plasmid DNA, for example, whether it exists in a more stable supercoiled form or in less stable open circular and linear forms. The conformation of the plasmid DNA can be determined by capillary electrophoresis or high-performance liquid chromatography (HPLC) of a sample containing the plasmid DNA. The content of supercoiled DNA can be measured under various conditions in comparison with open circular and linear forms.

In an embodiment of the present disclosure, at least 90% of the plasmid DNA is supercoiled. In some embodiments, at least 92.5% of the plasmid DNA in the liquid composition is supercoiled, but with no limitations thereto. In some embodiments, at least 95% of the plasmid DNA in the liquid composition is supercoiled. In some embodiments, at least 97% of the plasmid DNA in the liquid composition is supercoiled. In some embodiments, at least 98% of the plasmid DNA in the liquid composition is supercoiled.

In an embodiment of the present disclosure, the liquid composition further includes a buffer to maintain the pH of the pharmaceutical composition. The buffer may include buffering compounds known in the art, such as TAPS, Bicine, Tris, Tricine, TAPSO, HEPES, TES, MPOS, PIPES, Cacodylate, or MES. The buffer may contain citric acid, monopotassium phosphate, boric acid, or diethyl barbituric acid. The buffer may be a PBS, HEPES, TRIS, or TRIS/EDTA buffer. The buffer may be another phosphate buffer. Phosphate buffers may include a mixture of monobasic dihydrogen phosphate and dibasic monohydrogen phosphate.

The buffer may be a potassium phosphate buffer. The potassium phosphate buffer may contain potassium phosphate at a concentration of about 30-50 mM, about 35-50 mM, about 40-50 mM, about 45-50 mM, about 30-45 mM, about 35-45 mM, about 40-45 mM, about 30-40 mM, about 35-40 mM, or about 40 mM.

The buffer contained in the liquid composition may have a pH of about 7 to 9. In some embodiments, the pH is about 7 to 9, about 7.5 to 9, about 8 to 9, about 7 to 8.5, about 7.5 to 8.5, about 8 to 8.5, about 7 to 8, about 7.5 to 8, or about 8.

In an embodiment of the present disclosure, the liquid formulation may include about 30-50 mM potassium phosphate with a pH of about 7.5-8.5.

In an embodiment of the present disclosure, the liquid formulation further includes a salt. The salt may be NaCl. The NaCl may be included at a concentration of about 0.5-1.5%, about 0.5-1.4%, about 0.5-1.3%, about 0.5-1.2%, about 0.5-1.1%, about 0.5-1.0%, about 0.5-0.9%, about 0.6-1.5%, about 0.6-1.4%, about 0.6-1.3%, about 0.6-1.2%, about 0.6-1.1%, about 0.6-1.0%, about 0.6-0.9%, about 0.7-1.5%, about 0.7-1.4%, about 0.7-1.3%, about 0.7-1.2%, about 0.7-1.1%, about 0.7-1.0%, about 0.7-0.9%, about 0.8-1.5%, about 0.8-1.4%, about 0.8-1.3%, about 0.8-1.2%, about 0.8-1.1%, about 0.8-1.0%, about 0.8-0.9%, or 0.9%.

In an embodiment of the present disclosure, the liquid formulation includes about 40 mM potassium phosphate.

In an embodiment of the present disclosure, the liquid formulation includes about 0.9% NaCl.

In an embodiment of the present disclosure, the pH of the liquid formulation is about 8.0.

In an embodiment of the present disclosure, the liquid formulation includes about 40 mM potassium phosphate, about 0.9% NaCl, and has a pH of about 8.0. Herein, the liquid formulation according to one aspect of the present disclosure is referred to as Formulation A.

In an embodiment of the present disclosure, the plasmid in the liquid formulation maintains a supercoiled (SC) content of 80-100 %, a SC of 85-100%, a SC of 90-100%, or a SC of 95-100% after storage at 2-8°C for 3, 4, 5, or 6 months.

In an embodiment of the present disclosure, the plasmid in the liquid formulation maintains a SC of 80-100 %, 81-100 %, 82-100 %, 83-100 %, or 84-100 % after storage at 15-30°C for 3, 4, 5, or 6 months. The storage temperature may be 15-30°C, 16-30°C, 17-30°C, 18-30°C, 19-30°C, 20-30°C, 21-30°C, 22-30°C, 23-30°C, 24-30°C, 25-30°C, 15-28°C, 16-28°C, 17-28°C, 18-28°C, 19-28°C, 20-28°C, 21-28°C, 22-28°C, 23-28°C, 24-28°C, 25-28°C, 15-27°C, 16-27°C, 17-27°C, 18-27°C, 19-27°C, 20-27°C, 21-27°C, 22-27°C, 23-27°C, 24-27°C, 25-27°C, 15-26°C, 16-26°C, 17-26°C, 18-26°C, 19-26°C, 20-26°C, 21-26°C, 22-26°C, 23-26°C, 24-26°C, 25-26°C, or 25°C, but with no limitations thereto. The liquid formulation according to the present disclosure includes an aqueous solution containing a stabilizing amount of sugar to enhance the stability of plasmid DNA. The sugar may be sucrose, trehalose, mannitol, or sorbitol.

Therefore, the liquid formulation of the present disclosure may further contain sucrose, trehalose, mannitol, sorbitol, leucine, or a combination thereof.

In an embodiment of the present disclosure, the liquid formulation may further include about 1-2% of sucrose, about 0.5-2% of trehalose, about 0.01-1% of sorbitol, about 0.05-0.5% of leucine, or a combination thereof. In an embodiment of the present disclosure, the liquid formulation may include about 1.5% of sucrose, about 0.5-1.5% of trehalose, about 0.05-0.25% of sorbitol, about 0.1~0.5% of leucine, or a combination thereof.

A liquid formulation of plasmid DNA according to another aspect of the present disclosure includes mannitol in an amount of about 0.15 to 1.5% in addition to the liquid formulation described above according to an aspect of the present disclosure.

In an embodiment of the present disclosure, the liquid formulation includes mannitol at a concentration of about 0.15 to 1.5%, about 0.15 to 1.25%, about 0.15 to 1.0%, about 0.15 to 0.75%, about 0.15 to 0.5%, about 0.15 to 0.25%, about 0.25 to 1.5%, about 0.25 to 1.25%, about 0.25 to 1.0%, about 0.25 to 0.75%, about 0.25 to 0.5%, about 0.3 to 1.25%, about 0.3 to 1.0%, about 0.3 to 0.75%, about 0.3 to 0.5%, about 0.5 to 1.25%, about 0.5 to 1.0%, about 0.5 to 0.75%, about 1.5%, about 1.25%, about 1.0%, about 0.75%, about 0.5%, or about 0.25%, but with no limitations thereto.

Herein, the liquid formulation according to an aspect of the present disclosure is referred to as Formulation B.

In an embodiment of the present disclosure, the plasmid DNA in the liquid formulation maintains an SC of 90-100% after storage at 2-8°C for at least 3 months, 4 months, 5 months, or 6 months.

In an embodiment of the present disclosure, the plasmid DNA in the liquid formulation maintains an SC of 60-100%, 70-100%, 75-100%, 76-100%, 77-100%, 78-100%, 79-100%, or 80-100% SC% after storage at 15-30°C for at least 3 months, 4 months, 5 months, or 6 months. The storage temperature may be 15-30°C, 16-30°C, 17-30°C, 18-30°C, 19-30°C, 20-30°C, 21-30°C, 22-30°C, 23-30°C, 24-30°C, 25-30°C, 15-28°C, 16-28°C, 17-28°C, 18-28°C, 19-28°C, 20-28°C, 21-28°C, 22-28°C, 23-28°C, 24-28°C, 25-28°C, 15-27°C, 16-27°C, 17-27°C, 18-27°C, 19-27°C, 20-27°C, 21-27°C, 22-27°C, 23-27°C, 24-27°C, 25-27°C, 15-26°C, 16-26°C, 17-26°C, 18-26°C, 19-26°C, 20-26°C, 21-26°C, 22-26°C, 23-26°C, 24-26°C, 25-26°C, 15-25°C, 16-25°C, 17-25°C, 18-25°C, 19-25°C, 20-25°C, 21-25°C, 22-25°C, 23-25°C, 24-25°C, or 25°C, but with no limitations thereto.

A liquid formulation of plasmid DNA according to another aspect of the present disclosure includes about 0.001 to 0.3% of mannitol, about 0.05 to 0.5% of sorbitol, about 0.05 to 0.5% of sucrose, about 0.15 to 3% of trehalose, about 0.01 to 1% of leucine, or a combination thereof in addition to the liquid formulation described above.

Herein, the liquid formulation according to this aspect of the present disclosure is referred to as Formulation C.

In an embodiment of the present disclosure, the liquid formulation includes mannitol at a concentration of about 0.001 to 0.3%, about 0.005 to 0.3%, about 0.01 to 0.3%, about 0.02 to 0.3%, about 0.03 to 0.3%, about 0.04 to 0.3%, about 0.05 to 0.3%, about 0.001 to 0.25%, about 0.005 to 0.25%, about 0.01 to 0.25%, about 0.02 to 0.25%, about 0.03 to 0.25%, about 0.04 to 0.25%, about 0.05 to 0.25%, about 0.001 to 0.2%, about 0.005 to 0.2%, about 0.01 to 0.2%, about 0.02 to 0.2%, about 0.03 to 0.2%, about 0.04 to 0.2%, about 0.05 to 0.2%, about 0.001 to 0.15%, about 0.005 to 0.15%, about 0.01 to 0.15%, about 0.02 to 0.15%, about 0.03 to 0.15%, about 0.04 to 0.15%, about 0.05 to 0.15%, about 0.001 to 0.1%, about 0.005 to 0.1%, about 0.01 to 0.1%, about 0.02 to 0.1%, about 0.03 to 0.1%, about 0.04 to 0.1%, about 0.05 to 0.1%, about 0.001 to 0.075%, about 0.005 to 0.075%, about 0.01 to 0.075%, about 0.02 to 0.075%, about 0.03 to 0.075%, about 0.04 to 0.075%, about 0.05 to 0.075%, about 0.001 to 0.07%, about 0.005 to 0.07%, about 0.01 to 0.07%, about 0.02 to 0.07%, about 0.03 to 0.07%, about 0.04 to 0.07%, about 0.05 to 0.07%, about 0.001 to 0.06%, about 0.005 to 0.06%, about 0.01 to 0.06%, about 0.02 to 0.06%, about 0.03 to 0.06%, about 0.04 to 0.06%, about 0.05 to 0.06%, about 0.001 to 0.05%, about 0.005 to 0.05%, about 0.01 to 0.05%, about 0.02 to 0.05%, about 0.03 to 0.05%, about 0.04 to 0.05%, about 0.01%, about 0.03%, about 0.05%, about 0.07%, about 0.075%, or about 0.1%, but with no limitations thereto.

In an embodiment of the present disclosure, the liquid formulation includes sorbitol at a concentration of about 0.05 to 0.5%, about 0.075 to 0.5%, about 0.1 to 0.5%, about 0.125 to 0.5%, about 0.15 to 0.5%, about 0.2 to 0.5%, about 0.25 to 0.5%, about 0.3 to 0.5%, about 0.35 to 0.5%, about 0.4 to 0.5%, about 0.05%, 0.075%, about 0.1%, or about 0.15%, but with no limitations thereto.

In an embodiment of the present disclosure, the liquid formulation may include sucrose at a concentration of about 0.05% to 0.5%, about 0.075% to 0.5%, about 0.1% to 0.5%, about 0.125% to 0.5%, about 0.15% to 0.5%, about 0.2% to 0.5%, about 0.25% to 0.5%, about 0.3% to 0.5%, about 0.35% to 0.5%, about 0.4 to 0.5%, about 0.05%, about 0.075%, about 0.1%, about 0.15%, about 0.2%, or about 0.3%, but with no limitations thereto.

In an embodiment of the present disclosure, the liquid formulation includes trehalose at a concentration of about 0.15 to 3%, about 0.15 to 2.5%, about 0.15 to 2%, about 0.15 to 1.75%, about 0.15 to 1.5%, about 0.15 to 1.25%, about 0.15 to 1%, about 0.15 to 0.8%, about 0.15 to 0.75%, about 0.15 to 0.7%, about 0.15 to 0.6%, about 0.15 to 0.5%, about 0.25 to 3%, about 0.25 to 2.5%, about 0.25 to 2%, about 0.25 to 1.75%, about 0.25 to 1.5%, about 0.25 to 1.25%, about 0.25 to 1%, about 0.25 to 0.8%, about 0.25 to 0.75%, about 0.25 to 0.7%, about 0.25 to 0.6%, about 0.25 to 0.5%, about 0.5 to 3%, about 0.5 to 2.5%, about 0.5 to 2%, about 0.5 to 1.75%, about 0.5 to 1.5%, about 0.5 to 1.25%, about 0.5 to 1%, about 0.5 to 0.8%, about 0.5 to 0.75%, about 0.5 to 0.7%, about 0.5 to 0.6%, about 0.75 to 3%, about 0.75 to 2.5%, about 0.75 to 2%, about 0.75 to 1.75%, about 0.75 to 1.5%, about 0.75 to 1.25%, about 0.75 to 1%, about 0.75 to 0.8%, about 1 to 3%, about 1 to 2.5%, about 1 to 2%, about 1 to 1.75%, about 1 to 1.5%, about 1 to 1.25%, about 1.25 to 3%, about 1.25 to 2.5%, about 1.25 to 2%, about 1.25 to 1.75%, about 1.25 to 1.5%, about 1.5 to 3%, about 1.5 to 2.5%, about 1.5 to 2%, about 1.5 to 1.75%, 0.5%, about 1.0%, about 1.25%, about 1.5%, about 2%, about 2.5%, or about 3%, but with no limitations thereto.

In an embodiment of the present disclosure, the liquid formulation may include leucine at a concentration of about 0.01 to 1%, about 0.01 to 0.9%, about 0.01 to 0.8%, about 0.01 to 0.7%, about 0.01 to 0.6%, about 0.01 to 0.5%, about 0.01 to 0.4%, about 0.01 to 0.3%, about 0.01 to 0.2%, about 0.01 to 0.1%, about 0.03 to 1%, about 0.03 to 0.9%, about 0.03 to 0.8%, about 0.03 to 0.7%, about 0.03 to 0.6%, about 0.03 to 0.5%, about 0.03 to 0.4%, about 0.03 to 0.3%, about 0.03 to 0.2%, about 0.03 to 0.1%, about 0.05 to 1%, about 0.05 to 0.9%, about 0.05 to 0.8%, about 0.05 to 0.7%, about 0.05 to 0.6%, about 0.05 to 0.5%, about 0.05 to 0.4%, about 0.05 to 0.3%, about 0.05 to 0.2%, about 0.05 to 0.1%, about 0.075 to 1%, about 0.075 to 0.9%, about 0.075 to 0.8%, about 0.075 to 0.7%, about 0.075 to 0.6%, about 0.075 to 0.5%, about 0.075 to 0.4%, about 0.075 to 0.3%, about 0.075 to 0.2%, about 0.075 to 0.1%, about 0.08 to 1%, about 0.08 to 0.9%, about 0.08 to 0.8%, about 0.08 to 0.7%, about 0.08 to 0.6%, about 0.08 to 0.5%, about 0.08 to 0.4%, about 0.08 to 0.3%, about 0.08 to 0.2%, about 0.08 to 0.1%, about 0.09 to 1%, about 0.09 to 0.9%, about 0.09 to 0.8%, about 0.09 to 0.7%, about 0.09 to 0.6%, about 0.09 to 0.5%, about 0.09 to 0.4%, about 0.09 to 0.3%, about 0.09 to 0.2%, about 0.09 to 0.1%, about 0.01%, about 0.03%, about 0.05%, about 0.075%, about 0.08%, about 0.09%, about 0.1%, about 0.2%, or about 0.3%, but with no limitations thereto.

In an embodiment of the present disclosure, the liquid formulation may further include about 0.05% of mannitol, about 0.15% of sorbitol, about 0.3% of sucrose, about 1.5% of trehalose, about 0.1% of leucine, or a combination thereof.

In an embodiment of the present disclosure, the plasmid DNA in the liquid formulation maintains a SC of 90-100%, 91-100%, 92-100%, 93-100%, or 94-100% even after storage at 2-8°C for 3, 4, 5, 6, 7, 8, or 9 months.

In an embodiment of the present disclosure, the plasmid DNA in the liquid formulation maintains a SC of 80-100%, 81-100%, 82-100%, 83-100%, 84-100%, or 85-100% even after storage at 15-30°C for 3, 4, 5, 6, 7, 8, or 9 months. The storage temperature may be 15-30°C, 16-30°C, 17-30°C, 18-30°C, 19-30°C, 20-30°C, 21-30°C, 22-30°C, 23-30°C, 24-30°C, 25-30°C, 15-28°C, 16-28°C, 17-28°C, 18-28°C, 19-28°C, 20-28°C, 21-28°C, 22-28°C, 23-28°C, 24-28°C, 25-28°C, 15-27°C, 16-27°C, 17-27°C, 18-27°C, 19-27°C, 20-27°C, 21-27°C, 22-27°C, 23-27°C, 24-27°C, 25-27°C, 15-26°C, 16-26°C, 17-26°C, 18-26°C, 19-26°C, 20-26°C, 21-26°C, 22-26°C, 23-26°C, 24-26°C, 25-26°C, 15-25°C, 16-25°C, 17-25°C, 18-25°C, 19-25°C, 20-25°C, 21-25°C, 22-25°C, 23-25°C, 24-25°C, or 25°C, but with no limitations thereto.

In another aspect of the present disclosure, the liquid formulation of plasmid DNA may further include about 0.001 to 0.3% of mannitol, about 0.05 to 0.5% of sorbitol, about 0.05 to 0.5% of sucrose, about 0.15 to 3% of trehalose, about 0.045-0.055% of leucine, or a combination thereof.

In an embodiment of the present disclosure, the liquid formulation may include mannitol at a concentration of about 0.001 to 0.3%, about 0.005 to 0.3%, about 0.01 to 0.3%, about 0.02 to 0.3%, about 0.03 to 0.3%, about 0.04 to 0.3%, about 0.05 to 0.3%, about 0.001 to 0.25%, about 0.005 to 0.25%, about 0.01 to 0.25%, about 0.02 to 0.25%, about 0.03 to 0.25%, about 0.04 to 0.25%, about 0.05 to 0.25%, about 0.001 to 0.2%, about 0.005 to 0.2%, about 0.01 to 0.2%, about 0.02 to 0.2%, about 0.03 to 0.2%, about 0.04 to 0.2%, about 0.05 to 0.2%, about 0.001 to 0.15%, about 0.005 to 0.15%, about 0.01 to 0.15%, about 0.02 to 0.15%, about 0.03 to 0.15%, about 0.04 to 0.15%, about 0.05 to 0.15%, about 0.001 to 0.1%, about 0.005 to 0.1%, about 0.01 to 0.1%, about 0.02 to 0.1%, about 0.03 to 0.1%, about 0.04 to 0.1%, about 0.05 to 0.1%, about 0.001 to 0.075%, about 0.005 to 0.075%, about 0.01 to 0.075%, about 0.02 to 0.075%, about 0.03 to 0.075%, about 0.04 to 0.075%, about 0.05 to 0.075%, about 0.001 to 0.07%, about 0.005 to 0.07%, about 0.01 to 0.07%, about 0.02 to 0.07%, about 0.03 to 0.07%, about 0.04 to 0.07%, about 0.05 to 0.07%, about 0.001 to 0.06%, about 0.005 to 0.06%, about 0.01 to 0.06%, about 0.02 to 0.06%, about 0.03 to 0.06%, about 0.04 to 0.06%, about 0.05 to 0.06%, about 0.001 to 0.05%, about 0.005 to 0.05%, about 0.01 to 0.05%, about 0.02 to 0.05%, about 0.03 to 0.05%, about 0.04 to 0.05%, about 0.01%, about 0.03%, about 0.05%, about 0.07%, about 0.075%, or about 0.1%, but with no limitations thereto.

In an embodiment of the present disclosure, the liquid formulation may include potassium phosphate at a concentration of about 30-39 mM, about 31-39 mM, about 32-39 mM, about 33-39 mM, about 34-39 mM, about 34-39 mM, about 35-39 mM, about 36-39 mM, about 37-39 mM, about 30-38.5 mM, about 31-38.5 mM, about 32-38.5 mM, about 33-38.5 mM, about 34-38.5 mM, about 34-38.5 mM, about 35-38.5 mM, about 36-38.5 mM, about 37-38.5 mM, about 30-38 mM, about 31-38 mM, about 32-38 mM, about 33-38 mM, about 34-38 mM, about 34-38 mM, about 35-38 mM, about 36-38 mM, about 37-38 mM, about 30-37.5 mM, about 31-37.5 mM, about 32-37.5 mM, about 33-37.5 mM, about 34-37.5 mM, about 34-37.5 mM, about 35-37.5 mM, about 36-37.5 mM, about 37-37.5 mM, about 30-37.3 mM, about 31-37.3 mM, about 32-37.3 mM, about 33-37.3 mM, about 34-37.3 mM, about 34-37.3 mM, about 35-37.3 mM, about 36-37.3 mM, about 37-37.3 mM, about 36 mM, about 37 mM, about 37.3 mM, about 37.5 mM, about 38 mM, or about 39 mM.

In an embodiment of the present disclosure, the liquid formulation may include NaCl at a concentration of about 0.8-1.5%, about 0.85-1.5%, about 0.9-1.5%, about 0.95-1.5%, about 0.97-1.5%, about 0.975-1.5%, about 0.98-1.5%, about 0.8-1.25%, about 0.85-1.25%, about 0.9-1.25%, about 0.95-1.25%, about 0.97-1.25%, about 0.975-1.25%, about 0.98-1.25%, about 0.8-1.2%, about 0.85-1.2%, about 0.9-1.2%, about 0.95-1.2%, about 0.97-1.2%, about 0.975-1.2%, about 0.98-1.2%, about 0.8-1.1%, about 0.85-1.1%, about 0.9-1.1%, about 0.95-1.1%, about 0.97-1.1%, about 0.975-1.1%, about 0.98-1.1%, about 0.8-1.0%, about 0.85-1.0%, about 0.9-1.0%, about 0.95-1.0%, about 0.97-1.0%, about 0.975-1.0%, about 0.98-1.0%, about 0.8 %, about 0.9%, about 0.95%, or about 1.0%, but with no limitations thereto.

In an embodiment of the present disclosure, the liquid formulation may include about 37 mM of potassium phosphate, about 1.0% of NaCl, about 0.05% of mannitol, about 0.15% of sorbitol, about 0.3% of sucrose, about 1.5% of trehalose, about 0.05% of leucine, or a combination thereof, with a pH of about 8.0.

Herein, the liquid formulation according to an aspect of the present disclosure is referred to as formulation D.

In an embodiment of the present disclosure, the plasmid DNA within the liquid formulation maintains a SC of 90-100%, 91-100%, 92-100%, 93-100%, 94-100%, 95-100%, 95-100%, and 96-100% even after storage at 2-8°C for at least 3, 4, 5, 6, 7, 8, or 9 months.

In an embodiment of the present disclosure, the plasmid DNA within the liquid formulation maintains a SC of 80-100%, 81-100%, 82-100%, 83-100%, 84-100%, 85-100%, 86-100%, 87-100%, 88-100%, or 89-100% even after storage at 15-30°C for at least 3, 4, 5, 6, 7, 8, or 9 months. The storage temperature may be 15-30°C, 16-30°C, 17-30°C, 18- 30°C, 19-30°C, 20-30°C, 21-30°C, 22-30°C, 23-30°C, 24-30°C, 25-30°C, 15-28°C, 16-28°C, 17-28°C, 18-28°C, 19-28°C, 20-28°C, 21-28°C, 22-28°C, 23-28°C, 24-28°C, 25-28°C, 15-27°C, 16-27°C, 17-27°C, 18-27°C, 19-27°C, 20-27°C, 21-27°C, 22-27°C, 23-27°C, 24-27°C, 25-27°C, 15-26°C, 16-26°C, 17-26°C, 18-26°C, 19-26°C, 20-26°C, 21-26°C, 22-26°C, 23-26°C, 24-26°C, 25-26°C, 15-25°C, 16-25°C, 17-25°C, 18-25°C, 19-25°C, 20-25°C, 21-25°C, 22-25°C, 23-25°C, 24-25°C, or 25°C, but is not limited thereto.

In an embodiment of the present disclosure, the liquid formulation (Formulations A to D) according to one aspect of the present disclosure inhibit changes in the supercoiled percentage (SC%) of plasmid DNA.

In a specific embodiment of the present disclosure, the plasmid DNA SC% (supercoiled percentage) contained in the liquid formulation composition retains 80-100%, 85-100%, 90-100%, or 95-100% of the initial measurement after storage at 2-8°C for at least 3 months, 4 months, 5 months, or 6 months. For example, if the initial measurement of the plasmid DNA SC% contained in the liquid formulation composition of the present disclosure is 90 SC%, the plasmid DNA SC% measured again after 6 months at 2-8°C may range from 72 SC% to 90 SC%. The term "initial measurement" refers to the SC% of the plasmid DNA measured immediately after being incorporated into the liquid formulation of the present disclosure.

In another embodiment of the present disclosure, the plasmid DNA SC% (supercoiled percentage) contained in the liquid formulation composition retains 70-100%, 75-100%, 80-100%, 81-100%, 82-100%, 83-100%, 84-100%, or 85-100% of the initial measurement after storage at 15-30°C for at least 3 months, 4 months, 5 months, or 6 months.

For example, if the initial measurement of the plasmid DNA SC% contained in the liquid formulation composition of the present disclosure is 90 SC%, the plasmid DNA SC% measured again after 6 months at 30°C may range from 63 SC% to 90 SC%.

The storage temperature may be 15-30°C, 16-30°C, 17-30°C, 18-30°C, 19-30°C, 20-30°C, 21-30°C, 22-30°C, 23-30°C, 24-30°C, 25-30°C, 15-28°C, 16-28°C, 17- 28°C, 18-28°C, 19-28°C, 20-28°C, 21-28°C, 22-28°C, 23-28°C, 24-28°C, 25-28°C, 15-27°C, 16-27°C, 17-27°C, 18-27°C, 19-27°C, 20-27°C, 21-27°C, 22-27°C, 23-27°C, 24-27°C, 25-27°C, 15-26°C, 16-26°C, 17-26°C, 18-26°C, 19-26°C, 20-26°C, 21-26°C, 22-26°C, 23-26°C, 24-26°C, 25-26°C, 15-25°C, 16-25°C, 17-25°C, 18-25°C, 19-25°C, 20-25°C, 21-25°C, 22-25°C, 23-25°C, 24-25°C, or 25°C, but is not limited thereto.

In another aspect of the present disclosure, the liquid formulation is provided in unit dose form as a vial, ampule, bottle, or pre-filled syringe.

The liquid formulation according to an aspect of the present disclosure may be administered to a mammalian subject to treat various diseases. The liquid formulation of the present disclosure may be administered by various delivery methods, for example, orally or through parenteral routes, such as by intravenous, intramuscular, intraendocardial, intramyocardial, intrapericardial, intraventricular, intraarticular, intradermal, intracerebral, intrarenal, intrahepatic, intrasplenic, intralymphatic, subcutaneous, intraabdominal, intratesticular, intraovarian, intrauterine, sternal, intratracheal, intrapleural, intrathoracic, intradural, intraspinal, intramedullary, intramural, intrascorionic, arterial injection or infusion, or rectally, intranasally, by inhalation, or intraocularly. In certain embodiments, the delivery method may be achieved through intramuscular, intramyocardial, intravenous, intracerebral, or intrarenal routes.

In an embodiment of the present disclosure, the nucleic acid construct is administered by injection of the liquid pharmaceutical composition. In a specific embodiment, the polynucleotide construct is administered by intramuscular injection. Typically, the polynucleotide construct is administered by intramuscular injection near the affected area. In some embodiments, the polynucleotide construct is administered into the muscles of the subject's limbs, heart, or other body parts.

In some embodiments, the construct is injected subcutaneously or intradermally. In some embodiments, the polynucleotide construct is administered by intravascular delivery. In a specific embodiment, the construct is administered by retrograde intravenous injection.

It should be understood that the typical daily dosage of the composition of the liquid formulation of the present disclosure should be determined in light of various relevant factors, including the condition to be treated, the selected route of administration, the age, sex, and weight of the individual patient, and the severity of the patient's symptoms, and that it may be administered in a single dose or in divided doses. The polynucleotide construct is administered at a therapeutically effective dose.

In some embodiments of the methods described herein, the polynucleotide construct is administered at a total dosage of 1 µg to 200 mg, 1 mg to 200 mg, 1 mg to 100 mg, 1 mg to 50 mg, 1 mg to 20 mg, 5 mg to 10 mg, 16 mg, 8 mg, or 4 mg.

In a typical embodiment, the total dosage is divided into multiple individual injection doses. In some embodiments, the total dosage is divided into multiple equal injection doses. In some embodiments, the total dosage is divided into unequal injection doses.

In various embodiments of divided doses, the total dosage is administered at 4, 8, 16, 24, or 32 different injection sites.

In some embodiments, the injection dose is 0.1 to 5 mg. In a specific embodiment, the injection dose is 0.1 mg, 0.15 mg, 0.2 mg, 0.25 mg, 0.3 mg, 0.35 mg, 0.4 mg, 0.45 mg, or 0.5 mg.

The total dosage may be administered during a single visit or over two or more visits.

In a typical embodiment of divided doses, all multiple injection doses are administered within one hour of each other. In some embodiments, all multiple injection doses are administered within 1.5, 2, 2.5, or 3 hours of each other.

In various embodiments of the method, whether administered as a single unitary dose or divided into multiple injection doses, the total dosage of the polynucleotide construct is administered to the subject only once.

In some embodiments, the administration of the total dosage of the polynucleotide construct to multiple injection sites over one, two, three, or four visits may constitute a single cycle. Specifically, administration of 32 mg, 16 mg, 8 mg, or 4 mg of the polynucleotide construct to multiple injection sites over two visits may constitute a single cycle. The two visits may be spaced 3, 5, 7, 14, 21, or 28 days apart.

In some embodiments, the cycle may be repeated. The cycle may be repeated two, three, four, five, six, or more times.

In some embodiments, the cycle may be repeated after 1, 2, 3, 4, 5, 6, 7, 8, 9, 10, 11, 12 months or more after the previous cycle.

In some embodiments, the total dosage administered in the subsequent cycle may be the same as the total dosage administered in the previous cycle. In some embodiments, the total dosage administered in the subsequent cycle may differ from the total dosage administered in the previous cycle.

In a preferred embodiment, the nucleic acid construct is administered at a dosage of 8 mg per affected limb, divided evenly over multiple intramuscular injections and multiple visits, with each multiple injection during any single visit performed at separate injection sites. In a specific embodiment, the nucleic acid construct is administered at a dosage of 8 mg per affected limb, divided equally into a first dose of 4 mg per limb on day 0 and a second dose of 4 mg per limb on day 14, with each of the first and second doses further divided equally into multiple injection doses.

The actual amount and rate of administration and the time course of administration will vary depending on the nature and severity of the disease being treated. In a typical embodiment, the polynucleotide construct is administered in an amount effective to reduce symptoms of the disease, such as pain. In some embodiments, the amount is effective in reducing symptoms within one week of administration. In some embodiments, the amount is effective in reducing symptoms within 2 weeks, 3 weeks, or 4 weeks after administration.

Plasmid DNA may be administered simultaneously or sequentially, alone or in combination with other plasmid DNA, depending on the condition to be treated.

In some embodiments, the pharmaceutical composition of the liquid formulation includes plasmid DNA encoding human HGF. The pharmaceutical composition of the liquid formulation may be administered to treat various diseases, for example, diseases previously shown to be treatable by the administration of plasmid DNA. The plasmid DNA may encode a therapeutic gene such as human HGF. Examples of the disease include ischemic or hepatic diseases, coronary artery disease (CAD), amyotrophic lateral sclerosis (ALS), peripheral artery disease (PAD), diabetic ulcers, diabetic peripheral neuropathy (DPN), or neuropathies resulting from diseases, injuries, infections, or vitamin deficiencies, but are not limited thereto. For example, neuropathies may be caused by diabetes, vitamin deficiency, autoimmune diseases, genetic or hereditary disorders, amyloidosis, uremia, toxins or poisons, trauma or injury, or tumors, or may be idiopathic. The documents cited herein are incorporated by reference in their entirety.

### Advantageous Effects of Invention

The liquid formulation containing plasmid DNA according to the present disclosure exhibits excellent efficacy in terms of storage stability of the pharmaceutically active ingredient, plasmid DNA, and can be usefully employed as a pharmaceutical composition with superior stability and safety.

### Brief Description of Drawings

FIG. 1 is a diagram showing the results of long-term stability testing of Liquid Formulation A according to the present disclosure.
FIGS. 2a and 2b are diagrams showing the results of statistical analysis of the influence of various factors to derive Liquid Formulations B and C according to the present disclosure. (*KP = potassium phosphate, N = NaCl, MT = mannitol, SR = Sucrose, TH = Trehalose, LC = Leucine)
FIGS. 3a and 3b are diagrams showing the composition derived from statistical analysis to determine the optimal liquid formulation according to the present disclosure.
FIGS. 4 and 5 are diagrams showing the results of statistical analysis to derive the optimal liquid formulation for improving the long-term stability of plasmid DNA according to the present disclosure.
FIG. 6 is a diagram showing the comparison of plasmid DNA stability when the liquid formulation according to the present disclosure is prepared in vials and pre-filled syringes to verify whether there is any difference in storage stability based on unit dose form.

### Best Mode for Carrying out the Invention

### EXAMPLES

Throughout the specification, unless otherwise specified, "%" used to indicate the concentration of a specific substance refers to (weight/weight) % for solid/solid, (weight/volume) % for solid/liquid, and (volume/volume) % for liquid/liquid.

**TABLE 1**

| Abbreviated | |
|---|---|
| **AVE** | Average |
| **BDP** | Bulk Drug Product |
| **CMO** | Contract Manufacturing Organization |
| **DNA** | Deoxyribonucleic Acid |
| **DP** | Drug Product |
| **DS** | Drug Substance |
| **GMP** | Good Manufacturing Practice |
| **HX** | Helixmith Co., Ltd. |
| **N/A** | Not Applicable |
| **OC%** | Open-Circular pDNA Configuration |
| **pDNA** | Plasmid DNA |
| **SC%** | Supercoil pDNA Configuration |
| **ALD** | Company A |
| **WBU** | Company W |
| **CB** | Company C |

### EXAMPLE 1. Development of Liquid Formulation A Containing Plasmid DNA

Liquid Formulation A containing plasmid DNA according to the present disclosure was screened a total of five times. The component preparation, BDP manufacturing, vial filling, sampling, and SC% analysis used in the five rounds of screening were conducted in the same manner. The vials used in this example were washed according to the Vial Wash Protocol and stored in a 70°C dry oven up to 7 days before BDP formulation. Rubber stoppers were sterilized by the vendor (West-DAIKYO SEIKO) and used immediately after opening.

### BDP (Bulk Drug Product) Formulation

The 1X buffer and 2X buffer used in this example were prepared at least 7 days before the start of bulk drug product (BDP) manufacturing. The 2X buffer was prepared without NaCl, with double the buffer strength, and double the concentration of mannitol and sucrose (except that when the final formulation's NaCl concentration was 0.9%, the 2X buffer was also prepared with 0.9% NaCl, and when the final formulation's NaCl concentration was 1.1%, the 2X buffer was prepared with 1.3% NaCl).

The 1X buffer was identical in composition to the final formulation.

This test involved the preparation of a small-scale buffer (100 mL), and considering the manufacturing error of the actual commercial production process, the manufacturing error for 1X and 2X buffers was maintained within ~2%.

The API used in this test was a sample of VM202 DS (lot no. 88084) produced by Company A, which was thawed in a 2-8°C refrigerator for 20-24 hours after being transferred from a - 70°C deep freezer before use.

The thawed DS was mixed with 2X and 1X buffers to prepare BDP with a final pDNA concentration of 0.48-0.52 mg/mL (target 0.5 mg/mL).

The BDP manufacturing process, sampling, and analysis procedures are summarized in Table 2 below.

**TABLE 2**

| BDP (Bulk Drug Product) Manufacturing and Sampling protocols | |
|---|---|
| **No.** | **Procedure** |
| 1 | Prepare 2X and 1X buffers according to various formulations. |
| 2 | Turn on the BSC and prepare 2X, 1X, and DS samples. Thaw DS in the 2-8°C refrigerator the day before BDP formulation. |
| 3 | Add 20 mL of DS (Company A) to a Corning 150 mL storage bottle and record the current volume (A). |
| 4 | Add 20 mL of 2X buffer (B) and mix. |
| 5 | Mix for at least 5 minutes on a roller mixer and measure the total nucleic acid concentration using a NanoDrop (C). |
| 6 | Calculate the amount of 1X buffer to be added using the formula (((A+B) * (C/500)) - A - B) (D). |
| 7 | Add the calculated amount of 1X buffer in the BSC. |
| 8 | Mix for at least 2 minutes on a roller mixer and measure the total nucleic acid concentration three times using a NanoDrop (E) . |
| 9 | If the value of E satisfies the target range of 0.48 - 0.52 mg/mL, proceed to step 10. If E > 0.52, use the formula from step 6, substituting E for C and A+B+D for A+B, and calculate the additional volume of 1X buffer needed. Add the calculated amount of 1X buffer and measure the total nucleic acid concentration three times again. If D < 0.48, discard the sample and restart the BDP formulation from step 1. |
| 10 | Sample 500 µL of the completed BDP on Day 0 and store in a - 70°C deep freezer. |
| 11 | Dispense the remaining BDP into 20 mL vials, 5 mL each, and place in a 70°C oven. Record the time (F). |
| 12 | Sample 300 µL at the same time as F on Days 1, 3, 5, 7, and 10 in the BSC. |
| 13 | Store the sampled BDP in a -70°C deep freezer. |
| 14 | Thaw samples to be analyzed in a 2-8°C refrigerator 1 hour before SC% analysis. |
| 15 | Analyze SC% by HPLC and record the results. |

The list of formulations used for screening rounds 1 to 5 is shown in Tables 3 to 7 below.

**TABLE 3**

| List of Formulations Used in Screening Round 1 | | | | | | | | |
|---|---|---|---|---|---|---|---|---|
| Buffering agent, pH screening | | | | | | | | |
| No. | Formulation code | Buffer | pH | Strength (mM) | Excipient 1 (mannitol) | Excipient 2 (Sucrose) | Salt (NaCl) | API (mg/mL) |
| 1 | PA6.8 | potassium phosphate | 6.8 | 0.5 | 2% | 1% | 0.45% | 0.5 |
| 2 | PB6.8 | | | 1 | | | | |
| 3 | PC6.8 | | | 5 | | | | |
| 4 | PD6.8 | | | 10 | | | | |
| 5 | PA7.4 | | 7.4 | 0.5 | | | | |
| 6 | PB7.4 | | | 1 | | | | |
| 7 | PC7.4 | | | 5 | | | | |
| 8 | PD7.4 | | | 10 | | | | |
| 9 | PA8.0 | | 8.0 | 0.5 | | | | |
| 10 | PB8.0 | | | 1 | | | | |
| 11 | PC8.0 | | | 5 | | | | |
| 12 | PD8.0 | | | 10 | | | | |
| 13 | HAG.8 | Histidine | 6.8 | 0.5 | | | | |
| 14 | HB6.8 | | | 1 | | | | |
| 15 | HC6.8 | | | 5 | | | | |
| 16 | HD6. 8 | | | 10 | | | | |
| 17 | TR6.8 | Tris | 6.8 | 0.5 | | | | |
| 18 | TB6.8 | | | 1 | | | | |
| 19 | TC6.8 | | | 5 | | | | |
| 20 | TD6.8 | | | 10 | | | | |
| 21 | TA7.4 | | 7.4 | 0.5 | | | | |
| 22 | TB7.4 | | | 1 | | | | |
| 23 | TC7.4 | | | 5 | | | | |
| 24 | TD7.4 | | | 10 | | | | |
| 25 | TA8. 0 | | 8.0 | 0.5 | | | | |
| 26 | TB8.0 | | | 1 | | | | |
| 27 | TC8.0 | | | 5 | | | | |
| 28 | TD8. 0 | | | 10 | | | | |
| 29 | CA6.8 | Sodium Citrate | 6.8 | 0.5 | | | | |
| 30 | CB6.8 | | | 1 | | | | |
| 31 | CC6.8 | | | 5 | | | | |
| 32 | CD6.8 | | | 10 | | | | |
| 33 | CA7.4 | | 7.4 | 0.5 | | | | |
| 34 | CB7.4 | | | 1 | | | | |
| 35 | CC7.4 | | | 5 | | | | |
| 36 | CD7.4 | | | 10 | | | | |

**TABLE 4**

| List of Formulations Used in Screening Round 2 | | | | | | | | |
|---|---|---|---|---|---|---|---|---|
| Excipient Screening | | | | | | | | |
| No. | Formulation code | Buffer | pH | Strength (mM) | Excipient 1 (mannitol) | Excipient 2 (Sucrose) | Salt (NaCl) | API (mg/mL) |
| 1 | PMS0.1X | potassium phosphate | 8.0 | 5 | 0.2% | 0.1% | 0.45% | 0.5 |
| 2 | PMS0.5X | | | | 1% | 0.5% | | |
| 3 | PMS1X | | | | 2% | 1% | | |
| 4 | PMS2X | | | | 4% | 2% | | |
| 5 | PN45 | | | 40 | - | - | 0.45% | |
| 6 | P20/MS | | | 20 | 2% | 1% | 0.45% | |

**TABLE 5**

| List of Formulations Used in Screening Round 3 | | | | | | | |
|---|---|---|---|---|---|---|---|
| Salt Screening | | | | | | | |
| No. | Formulation code | Buffer | pH | Strength (mM) | Salt (NaCl) | Salt (MgCl₂) | API (mg/mL) |
| 1 | PN45 | potassium phosphate | 8.0 | 40 | 0.45% | - | 0.5 |
| 2 | PN45M | | | | 0.45% | 0.01% | |
| 3 | PN9 | | | | 0.9% | - | |
| 4 | PN9M1 | | | | 0.9% | 0.001% | |
| 5 | PN9M5 | | | | 0.9% | 0.005% | |

**TABLE 6**

| List of Formulations Used in Screening Round 4 | | | | | | |
|---|---|---|---|---|---|---|
| Double Buffer Screening | | | | | | |
| No. | Formulation code | Buffer | pH | Strength (mM) | Salt (NaCl) | API (mg/mL) |
| 1 | P40 | potassium phosphate (ctrl) | 8.0 | 40 | 0.9% | 0.5 |
| 2 | P40 | | | | | |
| 3 | PT3:1 | potassium phosphate, Tris | 8.0 | Phosphate 40mM, Tris 40mM | | |
| 4 | PT1:3 | | 8.0 | | | |
| 5 | PT8.1 | | 8.1 | | | |
| 6 | PT8.4 | | 8.4 | | | |
| 7 | PT8.7 | | 8.7 | | | |

Since the 4^{th} screening was conducted in two sessions, P40 control was prepared and analyzed for both sessions.

**TABLE 7**

| List of Formulations Used in Screening Round 5 (Long-term Stability Test) | | | | | | |
|---|---|---|---|---|---|---|
| Final candidates for the long-term stability test | | | | | | |
| No. | Formulation code | Buffer | pH | Strength (mM) | Salt (NaCl) | API (mg/mL) |
| 1 | P40 | potassium phosphate | 8.0 | 40 | 0.9% | 0.5 |
| 2 | P60 | | | 60 | | |
| 3 | P100 | | | 100 | 1.1% | |

### Screening Analysis Method

High temperature conditions accelerate the degradation of pDNA SC%, so in this example, Liquid DP vials were stored in a 70°C oven, and samples were taken on days 0, 1, 3, and 5 for SC% analysis (by HPLC). The sampled vials were stored in a -70°C deep freezer immediately after sampling, and HPLC analysis was performed after thawing the samples in a 2-8°C cold room for 1 hour before analysis.

To save time and cost on sample analysis, samples with a pDNA SC% below 10% were not analyzed for any subsequent samples taken after that point.

### Long-term Stability Study

To confirm the long-term stability of the liquid formulation, the inventors set conditions at 2-8°C, 25°C, and 37°C to monitor stability under various temperature conditions, and analyzed SC% at one-month intervals. Samples stored at 2-8°C were kept in an HX cold room, samples stored at 25°C were kept at room temperature, and samples stored at 37°C were kept in a 37°C incubator.

To maintain consistent sampling conditions, a maximum of four samplings were taken from each vial (5 mL filling), after which the final pH was measured and the vial was discarded.

Other conditions were identical to those used in the screening for rounds 1 to 4.

### Screening Analysis Results

### Example 1-1. Screening Round 1 (Buffering Agent, pH Screening)

The inventors sought to establish the type of buffering agent and pH range that could stably maintain VM202 pDNA.

First, to verify the effect of various buffering agents, potassium phosphate, Tris, histidine, and sodium citrate were used as buffering agents.

The final formulation list was selected by applying the possible pH range for each buffering agent. (However, the pH was set within a range that did not exceed pH 8.0, considering literature references such as Antoine Al-Achi, 2013.)

The SC% (by HPLC) analysis results for Screening Round 1 are shown in Table 8 below.

**TABLE 8**

| Results of Screening Round 1(Buffering agent, pH screening) | | | | | | |
|---|---|---|---|---|---|---|
| No. | Formulation code | SC% | | | | Final pH |
| | | Day 0 | Day 1 | Day 3 | Day 5 | |
| 1 | PA6.8 | 94 | 22 | | | 6.20 |
| 2 | PB6.8 | 94 | 35 | | | 6.43 |
| 3 | PC6.8 | 94 | 48 | | | 6.68 |
| 4 | PD6.8 | 94 | 39 | | | 6.63 |
| 5 | PA7.4 | 94 | 44 | | | 6.54 |
| 6 | PB7.4 | 94 | 60 | 4.2 | | 6.80 |
| 7 | PC7.4 | 94 | 75 | 11 | | 7.02 |
| 8 | PD7.4 | 94 | 69 | 11 | | 7.15 |
| 9 | PA8.0 | 94 | 56 | 4 | | 6.68 |
| 10 | PB8.0 | 94 | 68 | 7 | | 6.91 |
| 11 | PC8.0 | 94 | 74.4 | 35 | 5.3 | 7.30 |
| 12 | PD8.0 | 94 | 79 | 38 | 11 | 7.57 |
| 13 | HA6.8 | 94 | 0 | | | 6.91 |
| 14 | HB6.8 | 94 | 4.4 | | | 6.84 |
| 15 | HC6.8 | 94 | 6 | | | 6.52 |
| 16 | HD6.8 | 94 | 10 | | | 6.31 |
| 17 | TA6.8 | 94 | | | | 5.79 |
| 18 | TB6.8 | 94 | | | | 5.89 |
| 19 | TC6.8 | 94 | 6.4 | | | 6.19 |
| 20 | TD6.8 | 94 | 8.9 | | 2.6 | 6.56 |
| 21 | TA7.4 | 94 | | | | 6.14 |
| 22 | TB7.4 | 94 | | | | 6.04 |
| 23 | TC7.4 | 94 | 13.9 | | 10 | 7.28 |
| 24 | TD7.4 | 94 | 4.8 | | 4.1 | 7.49 |
| 25 | TA8.0 | 94 | | | | 6.02 |
| 26 | TB8.0 | 94 | 10 | | | 6.79 |
| 27 | TC8.0 | 94 | 41 | | 5.4 | 7.95 |
| 28 | TD8.0 | 94 | 32.9 | | 4.1 | 8.1 |
| 29 | CA6.8 | 94 | | | | 6.33 |
| 30 | CB6.8 | 94 | 59 | | | 6.40 |
| 31 | CC6.8 | 94 | 60 | 17 | | 6.76 |
| 32 | CD6.8 | 94 | 66 | | | 6.93 |
| 33 | CA7.4 | 94 | | | | 6.20 |
| 34 | CB7.4 | 94 | | | | 6.39 |
| 35 | CC7.4 | 94 | 37 | | | 6.59 |
| 36 | CD7.4 | 94 | 41 | | | 6.55 |

The SC% of VM202 pDNA tended to be higher as the pH approached 8.0, and it was most stable when potassium phosphate was used as the buffering agent.

Specifically, in the cases of PC8.0 and PD8.0, the SC% remained above 30% for 3 days. In PC7.4 and PD7.4, the SC% was maintained above 60% on day 1, but by day 3, the SC% had decreased to about 10%, indicating reduced SC% stability compared to PC8.0 and PD8.0.

Based on these results, it was confirmed that when using the same buffer, SC% stability was better maintained at higher pH levels.

It was also observed that higher buffer strength helped maintain pH over a longer period during formulation (refer to the results of PC8.0, PD8.0, TC8.0, and TD8.0). Therefore, it was confirmed that, with the same buffer, higher pH and buffer strength resulted in greater SC% stability.

The type of buffer also affected SC% stability, and it was confirmed that VM202 pDNA showed higher stability in potassium phosphate buffer and sodium citrate buffer.

Summarizing these results, the most suitable buffer for the VM202 liquid formulation was determined to be potassium phosphate buffer at pH 8.0.

### Example 1-2. Screening Round 2 (Excipient Screening)

Based on the results of the first screening (potassium phosphate, pH 8.0), various concentrations of mannitol and sucrose, which are used as stabilizers for pDNA, were tested to explore the optimal concentration range of excipients that contribute to pDNA stability.

The screening was conducted using the optimal buffering agent identified in the first screening, potassium phosphate at pH 8.0, as the base composition.

The stability of pDNA was compared by varying the concentrations of mannitol and sucrose.

The SC% (by HPLC) analysis results for screening round 2 are shown in Table 9 below.

**TABLE 9**

| Results of Screening Round 2 (Excipient screening ng) | | | | | | |
|---|---|---|---|---|---|---|
| No. | Formulation code | SC% | | | | Final pH |
| | | Day 0 | Day 1 | Day 3 | Day 5 | |
| 1 | PMS0.1 | 94 | 74.5 | 25.4 | 9.4 | 7.48 |
| 2 | PMS0.5 | 94 | 73.7 | 25.6 | 6.1 | 7.41 |
| 3 | PMS1 | 94 | 74.4 | 24 | 5.3 | 7.40 |
| 4 | PMS2 | 94 | 71 | 23 | 4.6 | 7.37 |
| 5 | PN45 | 95 | 73 | 48 | 12 | 7.84 |
| 6 | P20/MS | 95 | 73 | 17 | | 7.6 |

There was no significant difference in the SC% of pDNA over 5 days among PMS0.1, PMS0.5, PMS1, and PMS2. Additionally, in PN45 and P20/MS, both maintained SC% above 70% on day 1, but by day 3, PN45 showed more than 30% greater stability in SC% compared to P20/MS.

Based on this analysis, it was confirmed that the SC% of VM202 (non-GMP sample produced by Company A) was not affected by the concentration of mannitol and sucrose (refer to the results of PMS0.1 and PMS2).

Furthermore, since the Day 3 SC% of PN45 was approximately 30% higher than that of P20/MS, it was determined that buffer strength has a greater impact on pDNA SC% stability than the content of mannitol and sucrose (refer to the SC% results of PN45 and P20/MS).

Therefore, it was concluded that mannitol and sucrose could be removed from the formulation composition in Screening Round 3.

### Example 1-3. Screening Round 3 (Salt screening)

Based on the reference literature that suggests salts can stabilize pDNA by neutralizing the negative charge of the phosphate group of pDNA when depurination of pDNA occurs, the inventors sought to explore the optimal type and concentration range of salts that contribute to the stability of VM202's pDNA SC%.

Based on the results from the first and second screening rounds, the base composition used was 40 mM potassium phosphate, 0.45% NaCl, and pH 8.0.

Experiments were conducted by varying the concentrations of NaCl and MgCl₂, based on academic studies indicating that salts like NaCl and MgCl₂ affect pDNA stability.

Finally, considering the osmolality required for intramuscular injection, which is the intended route of administration for VM202, the concentration range of salts was set (ideally 280-360 mOsm/kg, preferably <600 mOsm/kg).

The SC% (by HPLC) analysis results for Screening Round 3 are shown in Table 10 below.

**TABLE 10**

| Results of Screening Round 3 (Salt screening) | | | | | | | |
|---|---|---|---|---|---|---|---|
| No. | Formulation code | Osmolality (mOsm/kg) | SC% | | | | Final pH |
| | | | Day 0 | Day 1 | Day 3 | Day 5 | |
| 1 | PN45 | 225 | 95 | 73 | 48 | 12 | 7.84 |
| 2 | PN45M | 226 | 95 | 77 | 57 | 29 | 7.78 |
| 3 | PN9 | 366 | 95 | 92 | 82 | 44 | 7.90 |
| 4 | PN9M1 | 366 | 95 | 89 | 77 | | 7.84 |
| 5 | PN9M5 | 367 | 95 | 86.5 | 72 | | 7.89 |

It was observed in VM202 pDNA that the stability of pDNA SC% increased with higher NaCl concentrations (refer to the results of PN45 and PN9).

Additionally, it was confirmed that the stability of pDNA SC% significantly increased when the NaCl concentration was 0.9%.

In the case of MgCl₂, when NaCl was at 0.45%, there was an approximately 10% increase in SC% stability, but over time, it was observed that MgCl₂ reacted with water to precipitate as Mg(OH)₂.

The maximum amount of MgCl₂ that could dissolve in 40 mM potassium phosphate and 0.9% NaCl was 0.01%; anything above this concentration resulted in precipitation.

On day 5, MgCl₂ had precipitated in PN9M1 and PN9M5, ending the HPLC analysis.

Based on this analysis, it was confirmed that SC% stability significantly increased with higher NaCl concentrations (refer to the results of PN45 and PN9).

Additionally, it was observed that MgCl₂, when in aqueous solution, began to precipitate as Mg(OH)₂ at concentrations above a certain level (PN45M conditions). This is likely due to the high pH of 8.0 used in this round, which, due to the high concentration of (OH)- ions, led to the formation of Mg(OH)₂. Therefore, it was confirmed that at salt concentrations above those in PN45M, Mg(OH)₂ formation prevented further improvement in pDNA SC% stability.

Summarizing these results, it was determined that adding NaCl at a concentration of 0.9% alone provided the highest pDNA SC% stability (Day 5 SC%: 44%). Based on the results up to the third screening round, the optimal composition for the liquid formulation of VM202 pDNA (Company A) was determined to be 40 mM potassium phosphate and 0.9% NaCl.

### Example 1-4. Screening Round 4 (Double Buffer Screening)

The inventors sought to verify whether the pDNA SC% would be more stable when using two buffering agents (double buffering) consisting of potassium phosphate and Tris, compared to using only one buffering agent, potassium phosphate. Based on the observation from Screening Rounds 1 to 3 that higher pH levels resulted in more stable pDNA, examination was made to see whether pDNA could be further stabilized by adjusting the pH upward using these two buffering agents.

First, the two buffering agents, phosphate buffer and Tris buffer, were mixed in different ratios from that used previously to prepare pDNA in liquid formulation form.

Based on the results from Screening Round 1, which indicated that higher pH levels led to greater pDNA stability, phosphate and Tris were mixed to achieve a pH higher than 8.0 and tested the stability of pDNA.

The NaCl concentration was fixed at 0.9%, referencing the results from Screening Round 3.

The SC% (by HPLC) analysis results for Screening Round 4 are shown in Table 11 below.

**TABLE 11**

| Results of Screening Round 4 (Double buffer screening) | | | | | | | |
|---|---|---|---|---|---|---|---|
| No. | Formulation code | pH (Day 0) | SC% | | | | Final pH |
| | | | Day 0 | Day 1 | Day 3 | Day 5 | |
| 1 | P40 | 8.00 | 94 | 92 | 82 | 44 | 8.07 |
| 2 | P40 | 8.03 | 94 | 81 | 60 | 54 | 8.02 |
| 3 | PT3:1 | 7.97 | 94 | 81 | 57 | 29 | 8.01 |
| 4 | PT1:3 | 8.02 | 94 | 66 | 14 | 5 | 8.03 |
| 5 | PT8.1 | 8.12 | 94 | 80 | 41 | 16 | 8.12 |
| 6 | PT8.4 | 8.36 | 94 | 82 | 54 | 36 | 8.33 |
| 7 | PT8.7 | 8.72 | 94 | 84 | 72 | 55 | 8.66 |

In the experiment where the pH was fixed at 8.0 and the ratio of Phosphate to Tris was varied, it was observed that the higher the proportion of phosphate, the more stable the pDNA was (refer to the results of P/T 3:1 and P/T 1:3). In the experiment where the ratio of phosphate to Tris was fixed and the pH was varied, it was confirmed that pDNA stability increased with higher pH levels.

Ultimately, when using a double buffer with a phosphate to Tris ratio of 1:1 at pH 8.7, the pDNA stability was similar to that of 40 mM phosphate at pH 8.0.

Through the fourth screening, it was confirmed that the higher the proportion of phosphate, the more the pDNA SC% stability was maintained over the long term.

It was also confirmed that when the ratio of phosphate to Tris was equal, pDNA was more stable at higher pH levels.

Therefore, in the case of a double buffer formulation, it was confirmed that increasing the proportion of phosphate buffer and the formulation pH leads to greater pDNA stability.

However, it was observed that the SC% stability of P40 and P/T8.7 were similar. This indicates that the effect of the double buffer formulation on pDNA SC% stability does not differ significantly from that of the existing single buffer formulation. Additionally, using a double buffer increases osmolarity because each buffer is used at a strength of 40 mM.

Therefore, based on these results, the existing single buffer with 40 mM phosphate at pH 8.0 is considered the most suitable.

### Example 1-5. Screening Round 5 (Long-term Stability of Final Candidates)

Through screening rounds 1 to 4, the selected formulation (40 mM phosphate, 0.9% NaCl, pH 8.0) and additional lead candidates were tested for long-term stability at various temperatures.

To determine the saturation point of the buffering agent and salt under the selected conditions of 40 mM phosphate, 0.9% NaCl, and pH 8.0, long-term stability studies were conducted with additional conditions of 60 mM phosphate, 0.9% NaCl / 100 mM phosphate, and 1.1% NaCl.

Samples were taken at various time points under storage conditions of 2-8°C (DP storage temperature), 25°C (accelerated conditions), and 37°C (stress conditions).

To maintain consistent sampling conditions, a maximum of four samplings were taken from each vial (5 mL filling), and the remaining sample was discarded after measuring the final pH.

The plan for the long-term stability study is detailed in Table 12 below.

**TABLE 12**

| Plan for Long-term Stability Study | | | | | | |
|---|---|---|---|---|---|---|
| Formulation code | Temperature Conditions (°C) | Time Point (month) | | | | |
| | | T0 | T1 | T2 | T3 | T6 |
| P40 | 2-8 | X | X | X | X | X |
| | 25 | X | X | X | X | X |
| | 37 | X | X | X | X | X |
| P60 | 2-8 | X | X | X | X | X |
| | 25 | X | X | X | X | X |
| | 37 | X | X | X | X | X |
| P100 | 2-8 | X | X | X | X | X |
| | 25 | X | X | X | X | X |
| | 37 | X | X | X | X | X |

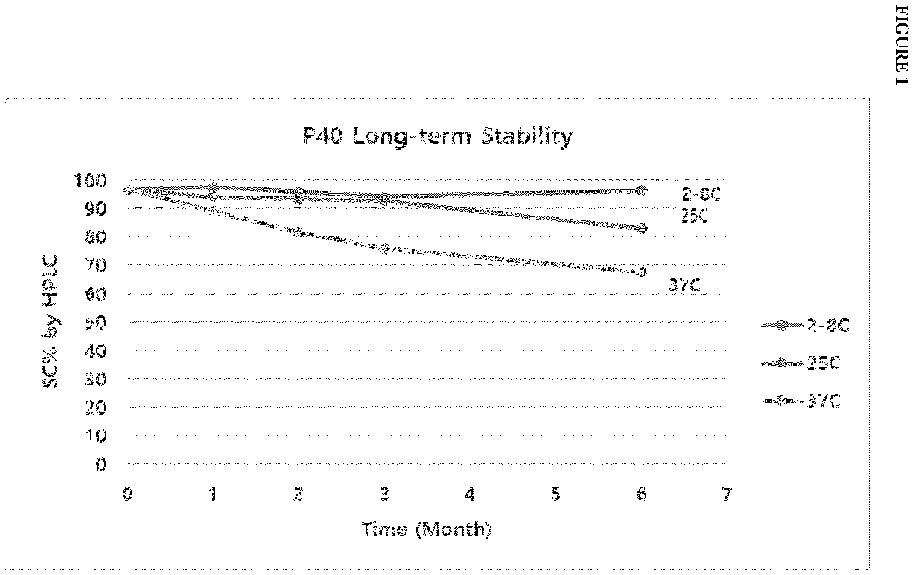

The SC% (by HPLC) analysis results for Screening Round 5 are presented in Table 13 and FIG. 1 below.

**TABLE 13**

| SC% Results for Screening Round 5 (Long-term Stability of Final Candidates) | | | | | | |
|---|---|---|---|---|---|---|
| Formulation code | Temperature Conditions (°C) | Time P oint ( month ) | | | | |
| | | T0 | T1 | T2 | T3 | T6 |
| P40 | 2-8 | 96.8 | 97.5 | 95.8 | 94.2 | 96.3 |
| | 25 | 96.8 | 94 | 93.2 | 89.5 | 83.0 |
| | 37 | 96.8 | 89 | 81.5 | 75.9 | 67.7 |
| P60 | 2-8 | N/A | 96.6 | N/A | N/A | N/A |
| | 25 | N/A | 94.4 | N/A | N/A | N/A |
| | 37 | N/A | 85 | N/A | 85.8 | N/A |
| P100 | 2-8 | N/A | N/A | N/A | N/A | N/A |
| | 25 | N/A | N/A | N/A | N/A | N/A |
| | 37 | N/A | 83.8 | N/A | N/A | 64.5 |

| | | | | | | |
|---|---|---|---|---|---|---|
| - N/A indicates that the analysis was not performed. | | | | | | |

It was observed that the compositions of P40, P60, and P100 all maintained similar SC% levels after storage under various temperature conditions.

For P40, the SC% was observed to remain 80% or higher after 6 months of storage at 25°C, exceeding the acceptance criteria for VM202 DP.

As predicted, it was concluded that this composition would remain stable for 18 months or longer when stored at 2-8°C, as the SC% after 6 months of storage at 2-8°C showed less than a 1% difference from the initial time point (T0).

Under the stress condition of 37°C, the SC% was maintained at around 80% for approximately 2 months.

From these results, it was confirmed that the SC% analysis results were similar across all conditions of P40, P60, and P100. This indicates that the VM202 pDNA SC% stability is maximized with a phosphate buffer strength of 40 mM, NaCl at 0.9%, and pH 8.0, and that increasing NaCl concentration or pH does not further influence SC% stability.

Therefore, based on these results, the P40 formulation (pH 8.0, 40 mM phosphate, 0.9% NaCl) is considered the most suitable as the final formulation for VM202 DP.

### Conclusion of Example 1

Through four rounds of screening and one round of 6-month stability testing, successful development was made of the VM202 liquid formulation that met the target stability (more than 80 SC% after 3 months at 25°C).

This example confirmed that not only pH but also the type of buffering agent affects the stability of VM202 pDNA. Although expected to maintain DNA stability better than NaCl based on academic literature, MgCl₂ precipitated as Mg(OH)₂, failing to contribute to DNA stability. This is likely due to the relatively high pH of 8.0 in the VM202 formulation, leading to the formation of Mg(OH)₂ due to the high concentration of (OH)⁻ ions, contrary to what was suggested in the literature.

Additionally, the 6-month stability test confirmed that the VM202 pDNA remained stable (83% SC) after 6 months of storage at 25°C with a composition of 40 mM phosphate buffer, 0.9% NaCl, and pH 8.0.

The final formulation will be subjected to additional long-term stability testing (18 months) at 2-8°C if necessary, and the resulting data will be used to determine the formulation range that can be applied in future commercial production.

The final formulation derived from the five screening rounds in this example is shown in Table 14 below.

**TABLE 14**

| Final Derived Formulation | | |
|---|---|---|
| **Ingredient** | **Concentration** | **Purpose** |
| VM202 DS | 0.5mg/mL | Active Pharmaceutical Ingredient |
| potassium phosphate | 40mM | Buffering Agent |
| NaCl | 0.9% | Stabilizer |
| To the final pH of 8.0 | | |

The final derived formulation (40 mM potassium phosphate, 0.9% NaCl, pH 8.0) demonstrated stability exceeding the DP specification after 3 months of storage at 25°C (89.5% SC), and it is expected to remain stable for over 18 months under 2-8°C storage conditions. Based on the final derived formulation, the range of formulations applicable to future commercial production will be identified.

### EXAMPLE 2: Development of Liquid Formulations B and C for Plasmid DNA

When applying the liquid formulation derived from Example 1 to VM202 DS produced by Companies C, W, and Helixmith, it was observed that the SC% stability differed from that of the VM202 DS produced by Company A. Therefore, this experiment was conducted to expand the limitations of the liquid formulation that was initially confined to VM202 DS produced by Company A. The liquid formulation applied to the VM202 DS produced by Company A in Example 1 was named Formulation A.

As in the previous example, the stability of VM202 DP was evaluated based on the SC% ratio, which is one of the factors determining the quality of VM202.

Assuming that the basic characteristics of VM202 DS are the same, the screening for this example was conducted based on the composition of Formulation A selected in Example 1.

Selection was made of Formulation B with reference to the screening results of Formulation A, and Formulation C, which had the optimal concentration of each excipient, through DoE (Design of Experiments).

The stability target for this screening was to maintain the DP Spec (SC% 85%) for more than 3 months under storage conditions of 20-25°C. This target was set based on studies indicating that the degradation of pDNA SC% accelerates as storage temperature increases, assuming that if stability is maintained for 3 months at 20-25°C, it would remain stable for over 18 months at the DP storage temperature of 2-8°C.

To shorten the development period, pDNA was exposed to 70°C for 5 days (accelerated conditions) to promote degradation, followed by SC% analysis. For the long-term stability test, samples were taken every 2 weeks (every month at 2-8°C) after storage at 2-8°C, 20-25°C, and 37°C for 3 months (with a 3-year plan for 2-8°C).

The four rounds of screening conducted in this example followed the same process as in Example 1, including component preparation, BDP manufacturing, vial filling, sampling, and SC% analysis.

The vials used in the test were washed according to the Vial Wash Protocol, stored in a 70°C dry oven, and used within 2 days before BDP formulation, following the actual production process as closely as possible. The rubber stoppers were sterilized by the vendor (West-DAIKYO SEIKO) and used immediately after opening.

### Preparation of Formulation

The 1X buffer and 2X buffer used in this example were prepared no more than three days before the start of BDP manufacturing for each round.

For the 2X buffer, the buffer strength, and the concentrations of mannitol, sucrose, sorbitol, trehalose, leucine, and arginine were doubled (however, the NaCl concentration was calculated based on the final NaCl concentration (A) using the following formula: *(A x 2) - 0.9 = NaCl concentration of the 2X buffer).

The 1X buffer was identical in composition to the final formulation.

This example involved the preparation of a small-scale buffer (100 mL), and considering the manufacturing tolerances of the actual commercial production process, the 1X and 2X buffers were prepared within a manufacturing error range of ~2%.

The API used in this example included samples of VM202 DS produced by Company A (lot no. 88084), Company C (lot no. 2017#0042U), Helixmith in-house production, and Company W (lot nos. DEV19-035, 049, 061, 068). These samples were thawed in a refrigerator at 2-8°C for 20-24 hours after being transferred from a -70°C deep freezer.

The thawed DS was mixed with the 2X and 1X buffers to produce BDP with a final pDNA concentration of 0.48-0.52 mg/mL (Target 0.5 mg/mL).

The BDP manufacturing process, as well as the sampling and analysis protocols, are shown in Table 15 below.

**TABLE 15**

| BDP Manufacturing and Sampling Protocols | |
|---|---|
| No. | Procedure |
| 1 | Prepare 2X and 1X buffers according to the formulation lists in Tables 7-12. |
| 2 | Turn on the BSC and prepare the 2X, 1X, and DS samples-thaw the DS in a refrigerator at 2-8°C the day before BDP formulation. |
| 3 | Add 5-7 mL of DS to a SPL 50 mL conical tube and record the current volume (A). |
| 4 | Add an equal volume of 2X buffer (B) to the DS and mix. |
| 5 | Mix for at least 5 minutes on a roller mixer, then measure the total nucleic acid concentration using a NanoDrop (C). |
| 6 | Calculate the amount of 1X buffer to add using the formula (((A+B) * (C/500) - A-B)). |
| 7 | Add the calculated amount of 1X buffer in the BSC. |
| 8 | Mix for at least 2 minutes on a roller mixer, then measure the total nucleic acid concentration three times using a NanoDrop (E). |
| 9 | If E is within the range of 0.48-0.52 mg/mL, proceed to step 10. If E > 0.52, recalculate the 1X buffer volume to add using the formula with E instead of C and A+B+D instead of A+B in No. 6, then add the additional 1X buffer and measure the total nucleic acid concentration three times. |
| | If D < 0.48, discard the sample and restart from BDP formulation step 1. |
| 10 | Adjust the pH to 8.0, then filter the BDP using a vacuum filter (applied from DOE onwards). |
| 11 | Sample 500 µL from the prepared sample on Day 0 and store it in a -70°C deep freezer. |
| 12 | Dispense the remaining sample into 20 mL vials, with 5 mL in each vial, then place them in a 70°C oven and record the time (F). |
| 13 | On Days 1, 3, and 5, sample 200 µL at the same time as F in the BSC. |
| 14 | Store the sampled material in a -70°C deep freezer. |
| 15 | Thaw the sample in a 2-8°C refrigerator 1 hour before SC% analysis. |
| 16 | Analyze SC% (by HPLC) and record the results. |

The formulation lists used in Screening Rounds 1 to 4 are shown in Tables 16 to 21 below.

**TABLE 16**

| Round 1 Pre-screening List 1 (Excipients) | | | | | | | |
|---|---|---|---|---|---|---|---|
| No. | DS source | Formulation code | pH | K phosphate (mM) | Salt (NaCl) | Excipient (%) | API (mg/mL) |
| 1 | Company C | CBCON | 8.0 | 40 | 0.9% | - | 0.5 |
| 2 | | M05 | | | | mannitol (0.5) | |
| 3 | | M15 | | | | mannitol (1.5) | |
| 4 | | S05 | | | | sucrose (0.5) | |
| 5 | | S15 | | | | sucrose (1.5) | |
| 6 | | SB05 | | | | sorbitol (0.5) | |
| | | SB15 | | | | sorbitol (1.5) | |
| 7 8 | | T05 | | | | trehalose (0.5) | |
| 9 | | T15 | | | | trehalose (1.5) | |
| 10 | | L025 | | | | leucine (0.25) | |
| 11 | | L05 | | | | leucine (0.5) | |
| 12 | | A005 | | | | arginine (0.05) | |
| 13 | ALD | ALCON | | | | - | |

Since potassium phosphate was selected as the optimal buffer for liquid formulation development in the previous example related to Formulation A, the buffer was fixed as potassium phosphate in this example.

**TABLE 17**

| Round 1 Pre-screening List 2 (arginine) | | | | | | | | |
|---|---|---|---|---|---|---|---|---|
| No. | DS source | Formulation code | K phosphate (mM) | pH | Salt (NaCl) | Excipient 1 (%) | Excipient 2 (arginine) | API (mg/mL) |
| 1 | Company C | CON | 40 | 8.0 | 0.38% | - | - | 0.5 |
| 2 | | M025 | | | | mannitol (0.25) | - | |
| 3 | | M025A | | | | mannitol (0.25) | 0.0025% | |
| 4 | | S025 | | | | sucrose (0.25) | - | |
| 5 | | S025A | | | | sucrose (0.25) | 0.0025% | |
| 6 | | T025 | | | | trehalose (0.25) | - | |
| 7 | | T025A | | | | trehalose (0.25) | 0.0025% | |
| 8 | | SB025 | | | | sorbitol (0.25) | - | |
| 9 | | SB025A | | | | sorbitol (0.25) | 0.0025% | |
| 10 | | L025 | | | | leucine (0.25) | - | |
| 11 | | L025A | | | | leucine (0.25) | 0.0025% | |

**TABLE 18**

| Round 1 Pre-screening List 3 (Range) | | | | | | | |
|---|---|---|---|---|---|---|---|
| No . | DS Source | Formulation code | K phosphate (mM) | pH | Salt (NaCl) | Excipient | API (mg/mL) |
| 1 | Company C | CON | 40 | 8.0 | 0.9% | - | 0.5 |
| 2 | | M025 | | | | mannitol 0.25% | |
| 3 | | S025 | | | | sucrose 0.25% | |
| 4 | | T025 | | | | trehalose 0.25% | |
| 5 | | SB025 | | | | sorbitol 0.25% | |
| 6 | | L025 | | | | leucine 0.25% | |

**TABLE 19**

| Round 2 Design of Experiment (DoE) | | | | | | | | | |
|---|---|---|---|---|---|---|---|---|---|
| DOE; Random Design | | | | | | | | | |
| No. | K phosphate (mM) | Salt (NaCl) (%) | pH | Excipient (%) | | | | | API (mg/mL) |
| | | | | mannitol | sucro se | sorbitol | trehalose | leucine | |
| 1 | 20 | 0.45 | 7.8 | 0.15 | 0.25 | 0.3 | 1.5 | 0.5 | 0.5 |
| 2 | 20 | 0.45 | 7.8 | 0.15 | 0.25 | 0.3 | 1.5 | 0.5 | |
| 3 | 20 | 0.9 | 7.9 | 0.25 | 0.05 | 0.3 | 0.3 | 0.5 | |
| 4 | 20 | 0.9 | 7.9 | 0.25 | 0.05 | 0.3 | 0.3 | 0.5 | |
| 5 | 40 | 0.45 | 7.9 | 0.05 | 0.25 | 1.5 | 0.5 | 0.1 | |
| 6 | 40 | 0.45 | 7.9 | 0.05 | 0.25 | 1.5 | 1.5 | 0.1 | |
| 7 | 20 | 0.9 | 7.8 | 0.05 | 0.25 | 1.5 | 0.3 | 0.3 | |
| 8 | 20 | 0.9 | 7.8 | 0.05 | 0.25 | 1.5 | 0.3 | 0.3 | |
| 9 | 30 | 0.675 | 7.9 | 0.15 | 0.15 | 0.9 | 0.9 | 0.3 | |
| 10 | 30 | 0.675 | 7.9 | 0.15 | 0.15 | 0.9 | 0.9 | 0.3 | |
| 11 | 20 | 0.9 | 8 | 0.05 | 0.15 | 0.3 | 1.5 | 0.1 | |
| 12 | 20 | 0.9 | 8 | 0.05 | 0.15 | 0.3 | 1.5 | 0.1 | |
| 13 | 40 | 0.9 | 7.8 | 0.25 | 0.25 | 0.3 | 0.9 | 0.1 | |
| 14 | 40 | 0.9 | 7.8 | 0.25 | 0.25 | 0.3 | 0.9 | 0.1 | |
| 15 | 40 | 0.45 | 8 | 0.25 | 0.05 | 0.3 | 1.5 | 0.3 | |
| 16 | 40 | 0.45 | 8 | 0.25 | 0.05 | 0.3 | 1.5 | 0.3 | |
| 17 | 40 | 0.9 | 8 | 0.15 | 0.05 | 1.5 | 0.3 | 0.1 | |
| 18 | 40 | 0.9 | 8 | 0.15 | 0.05 | 1.5 | 0.3 | 0.1 | |
| 19 | 40 | 0.9 | 7.8 | 0.05 | 0.05 | 0.9 | 1.5 | 0.5 | |
| 20 | 40 | 0.9 | 7.8 | 0.05 | 0.05 | 0.9 | 1.5 | 0.5 | |
| 21 | 30 | 0.9 | 8 | 0.25 | 0.25 | 1.5 | 1.5 | 0.5 | |
| 22 | 30 | 0.9 | 8 | 0.25 | 0.25 | 1.5 | 1.5 | 0.5 | |
| 23 | 40 | 0.45 | 7.8 | 0.25 | 0.15 | 1.5 | 0.3 | 0.5 | |
| 24 | 40 | 0.45 | 7.8 | 0.25 | 0.15 | 1.5 | 0.3 | 0.5 | |
| 25 | 20 | 0.675 | 7.8 | 0.25 | 0.05 | 1.5 | 1.5 | 0.1 | |
| 26 | 20 | 0.675 | 7.8 | 0.25 | 0.05 | 1.5 | 1.5 | 0.1 | |
| 27 | 40 | 0.675 | 8 | 0.05 | 0.25 | 0.3 | 0.3 | 0.5 | |
| 28 | 40 | 0.675 | 8 | 0.05 | 0.25 | 0.3 | 0.3 | 0.5 | |
| 29 | 20 | 0.45 | 8 | 0.25 | 0.25 | 0.9 | 0.3 | 0.1 | |
| 30 | 20 | 0.45 | 8 | 0.25 | 0.25 | 0.9 | 0.3 | 0.1 | |
| 31 | 20 | 0.45 | 8 | 0.05 | 0.05 | 1.5 | 0.9 | 0.5 | |
| 32 | 20 | 0.45 | 8 | 0.05 | 0.05 | 1.5 | 0.9 | 0.5 | |
| 33 | 30 | 0.45 | 7.8 | 0.05 | 0.05 | 0.3 | 0.3 | 0.1 | |
| 34 | 30 | 0.45 | 7.8 | 0.05 | 0.05 | 0.3 | 0.3 | 0.1 | |

**TABLE 20**

| Round 3 Long-Term Stability Test and Comparative Formulation List A | | | | | | | | | | | |
|---|---|---|---|---|---|---|---|---|---|---|---|
| Long-term Stability Study of Formulation B, Formulation C & Comparison Formulation A | | | | | | | | | | | |
| Formulat ion Type | DS source | Formula tion Code | K phosp hate (mM) | pH | NaCl (%) | mannit ol | sucro se | sorbit ol | trehalo se | leuci ne | API (mg/m 1) |
| C | HX | HX1C | 40 | 8.0 | 0.9 | 0.05% | 0.3% | 0.15% | 1.5% | 0.1% | 0.5 |
| | | HX2C | | | | | | | | | |
| | Compan y W | 1C from Company W | | | | | | | | | |
| | | 2C from Company W | | | | | | | | | |
| | ALD | ALDC | | | | | | | | | |
| B | HX | HX1B | 40 | 8.0 | 0.9 | 0.25% | - | - | - | - | |
| | | HX2B | | | | | - | - | - | - | |
| | Compan y W | 1B from Company W | | | | | - | - | - | - | |
| | | 2B from Company W | | | | | - | - | - | - | |
| | ALD | ALDB | | | | | - | - | - | - | |
| A | HX | HX1A | 40 | 8.0 | 0.9 | - | - | - | - | - | |
| | | HX2A | | | | - | - | - | - | - | |
| | Compan y W | 1A from Company W | | | | - | - | - | - | - | |
| | | 2A from Company W | | | | - | - | - | - | - | |
| | ALD | ALDA | | | | - | - | - | - | - | |

**TABLE 21**

| Round 4 Scale-Up Process Adjustment | | | | | | | | | | | | |
|---|---|---|---|---|---|---|---|---|---|---|---|---|
| Scale-up process adjustment | | | | | | | | | | | | |
| Formul ation Type | DS sourc e | Formu latio n Code | K phosp hate (mM) | pH | NaCl (%) | mannit ol | sucro se | sorbi tol | trehal ose | leuci ne | Filt er O/X | API (mg/m l) |
| C | HX | HXCW | 40 | 8.0 | 0.9 | 0.05% | 0.3% | 0.15% | 1.5% | 0.1% | O | 0.5 |
| | | HXCWO | | | | | | | | | X | |
| A | HX | HXAW | 40 | 8.0 | 0.9 | - | - | - | - | - | O | |
| | | HXAWO | | | | | | | | | X | |

After BDP manufacturing, the pH was adjusted to 8.0, and filtering was performed using Supor EKV-Mini Kleenpak Capsules filter (Pall Co., LTD), followed by aliquoting into vials.

### Analysis

Since high temperatures accelerate pDNA SC% degradation, in this test, the Liquid DP vials were stored in a 70°C oven, and samples were taken on day 0 and day 5 for SC% analysis by HPLC. (For Round 3, samples were taken on days 0, 1, 3, and 5.)

The analysis samples were stored in a -70°C deep freezer immediately after sampling, and were thawed in a 2-8°C cold room 1 hour before HPLC analysis.

To save time and cost on sample analysis, no further analysis was conducted on samples where the pDNA SC% was below 10%.

### Long-term Stability Study

To confirm long-term stability under various temperature conditions, samples were taken at 2-week intervals (1 month for 2-8°C) under the conditions of 2-8°C, 20-25°C, and 37°C, and SC% was analyzed.

Samples at 2-8°C were stored in the HX cold room, at 25°C in the 530 laboratory at room temperature, and at 37°C in a 37°C incubator.

To maintain consistent sampling conditions, a maximum of 200 µL was sampled from each vial (5 mL filling) up to 5 times before the vial was discarded.

### Scale-up Process Adjustment Study

The pDNA SC% stability was verified by scaling up from a small scale (~15 mL BDP) process to a large scale (150 mL BDP) process. The method for verifying SC% stability was the same as in Example 1.

To determine whether the presence or absence of a filter process affects the pDNA SC% stability in the compositions of Formulations A and C, experiments were conducted with and without the use of a filter after BDP manufacturing.

### Example 2-1. Screening Round 1 (Excipient)

Considering that the efficacy of the liquid formulation A established in Example 1 was limited to the DS produced by Company A, this study aimed to develop a formulation that exhibits similar stability to liquid formulation A + Company A's DS, but applicable to VM202 DS produced by other CMOs, such as Company C and Company W. To this end, various excipients were added to determine the types and concentration ranges of excipients that could enhance the SC% stability of VM202 pDNA produced by different manufacturers other than Company A.

### Study Design

To assess the effects of various excipients, mannitol, sucrose, sorbitol, trehalose, leucine, and arginine were treated at appropriate concentrations to identify significant ranges.

The concentration ranges of each excipient were selected based on research on components of biosimilars approved by the FDA (see Reference) and literature sources (Rev Bras Otorrinolaringol, 2006;72(3):400-6).

### Results

The osmolality and SC% (by HPLC) analysis results from pre-screenings 1 to 3 in the first round are shown in Tables 22 to 24 below.

**TABLE 22**

| Results of Pre-screening Round 1 (Excipients) | | | | | | |
|---|---|---|---|---|---|---|
| No. | Formulation code | Initial pH | Osmolality (mOsm/kg) | SC% | | Final pH |
| | | | | Day 0 | Day 5 | |
| 1 | CON | 7.96 | 367 | 97.1 | 21.4 | 7.96 |
| 2 | M05 | 7.97 | 388 | | 51 | 7.82 |
| 3 | M15 | 7.93 | 438 | | 50.5 | 7.77 |
| 4 | S05 | 7.95 | 376 | | 33 | 7.94 |
| 5 | S15 | 7.96 | 406 | | 39.4 | 7.92 |
| 6 | SB05 | 7.95 | 384 | 97.2 | 15 | 7.77 |
| 7 | SB15 | 7.95 | 434 | | 11.3 | 7.72 |
| 8 | T05 | 7.93 | 373 | | 41.1 | 7.94 |
| 9 | T15 | 7.93 | 396 | 96.8 | 44.5 | 7.92 |
| 10 | L025 | 7.96 | 383 | | 40 | 7.95 |
| 11 | L05 | 7.96 | 401 | | 30.2 | 7.97 |
| 12 | A005 | 8.64 | 369 | 96.9 | 51.2 | 8.53 |
| 13 | ALDCON | 7.97 | 367 | 96.2 | 35.1 | 7.98 |

Considering the overlap in excipient concentrations across pre-screenings 1 to 3, osmolality was measured only in pre-screening 1.

The SC% of VM202 pDNA showed a tendency to increase when mannitol was included (with similar results at both 0.5% and 1.5%), as well as with higher concentrations of sucrose and trehalose, or at higher pH levels (see A005 results).

**TABLE 23**

| Results of Round 1 Pre-screening 2 (arginine) | | | | | |
|---|---|---|---|---|---|
| No. | Formulation code | Initial pH | SC% | | Final pH |
| | | | Day 0 | Day 5 | |
| 1 | CON | 8.07 | 96.5% | 5.1 | 8.02 |
| 2 | M025 | 8.07 | | 30.9 | 7.92 |
| 3 | M025A | 8.07 | | 30.7 | 7.95 |
| 4 | S025 | 8.03 | | 9.9 | 8 |
| 5 | S025A | 8.07 | 97.3 | 10.2 | 8.02 |
| 6 | T025 | 8.04 | | 10.8 | 8 |
| 7 | T025A | 8.07 | | 11 | 8.03 |
| 8 | SB025 | 8.05 | | 16.3 | 7.9 |
| 9 | SB025A | 8.08 | 96.8 | 14.6 | 7.92 |
| 10 | L025 | 8.05 | | 18.5 | 8.01 |
| 11 | L025A | 8.07 | | 18.1 | 8 |

In Pre-screening 2, the NaCl concentration was set at 0.38%. The purpose of this Pre-screening 2 was to determine whether the increase in SC% stability observed in A005 during Pre-screening 1 was due to the pH increase caused by arginine or the effect of arginine itself.

To this end, two groups were created: one with additional arginine (0.0025%) added to the existing excipients and one without. The pH of both groups was adjusted to 8.0 (to eliminate the effect of pH increase), aiming to determine if arginine itself had an SC% stabilizing effect. The results showed no increase in SC% stability due to the addition of arginine.

It was confirmed that the increase in SC% stability observed in A005 during Pre-screening 1 was due to the pH increase, and not due to arginine itself. Therefore, arginine was excluded from the formulation in subsequent tests.

**TABLE 24**

| Results of Round 1 Pre-screening 3 (Range) | | | | | |
|---|---|---|---|---|---|
| No. | Formulation code | Initial pH | SC% | | Final pH |
| | | | Day 0 | Day 5 | |
| 1 | CON | 7.91 | 96.5 | 6.5 | 7.91 |
| 2 | M025 | 7.9 | | 35.3 | 7.83 |
| 3 | S025 | 7.91 | | 12.3 | 7.9 |
| 4 | T025 | 7.9 | | 14.7 | 7.91 |
| 5 | SB025 | 7.9 | | 19.8 | 7.8 |
| 6 | L025 | 7.91 | | 22.7 | 7.92 |

In Pre-screening 3, to assess stability under more stringent conditions, the test was conducted at pH 7.9. Compared to the control formulation (Formulation A), M025 showed the highest increase in SC%, and other excipients also demonstrated significant increases in SC% stability.

### Review

Among the added excipients, mannitol showed the highest increase in SC%, and it was confirmed that the amount of increase in SC% was similar across mannitol concentrations from 0.25% to 1.5%. Assuming the effect of mannitol saturates at 0.25%, the concentration range for mannitol in the DoE experiment was set to 0.05-0.25%.

Additionally, selection was made of Liquid Formulation B prepared by adding 0.25% mannitol to the existing Liquid Formulation A, to compare with the new Formulation C to be developed through the DoE.

The concentration range for the remaining excipients was also set based on their saturation points (maximum 1.5%). For sorbitol and leucine, the concentration range was set to be 0.05-0.25% and 0.1-0.5%, respectively, considering that lower concentrations resulted in higher SC%.

Furthermore, it was confirmed that pH buffering was more stable with disaccharides like sucrose and trehalose compared to monosaccharides like mannitol and sorbitol.

Based on these results, it was confirmed that by adding additional excipients to the existing Formulation A, which was limited to Company A's VM202 DS, it is possible to develop a new formulation applicable to VM202 DS produced by other manufacturers such as Company C and Company W. Additionally, the concentration ranges for each excipient affecting SC% stability were established to maximize the efficiency of the DoE experiments.

### Example 2-2. Second Round Experiment Design (Design of Experiment, DoE)

Using the excipient candidates and their concentration ranges selected in the first pre-screening, the optimal excipient composition that stabilizes VM202 pDNA was determined through a DoE approach.

### Experiment Design

Based on the effects of various excipients on pDNA stability observed in the first excipient screening, different combinations of concentration ranges were tested to evaluate their interactions and identify key excipient factors that influence pDNA stability.

The DoE was randomly designed and conducted in duplicate.

The excipients evaluated through the DoE were as follows:
mannitol, sucrose, sorbitol, trehalose, leucine

The results of SC% analysis (by HPLC) for the second round of DoE are shown in Table 25 below.

**TABLE 25**

| Round 2 Design of Experiment | | |
|---|---|---|
| DoE No. | SC (%) | |
| | Day | Day 5 |
| 1 | 98.1 | 4.2 |
| 2 | | 4.0 |
| 3 | | 8.2 |
| 4 | | 9.4 |
| 5 | | 10.6 |
| 6 | | 11.9 |
| 7 | | 4.4 |
| 8 | | 6.8 |
| 9 | | 5.3 |
| 10 | | 7.7 |
| 11 | | 18.4 |
| 12 | | 17.0 |
| 13 | | 19.4 |
| 14 | 97.2 | 14.4 |
| 15 | | 10.0 |
| 16 | | 10.3 |
| 17 | | 18.5 |
| 18 | | 20.0 |
| 19 | | 7.0 |
| 20 | | 7.2 |
| 21 | | 6.5 |
| 22 | | 6.8 |
| 23 | | 4.8 |
| 24 | | 5.6 |
| 25 | | 14.9 |
| 26 | | 10.7 |
| 27 | | 10.7 |
| 28 | | 9.4 |
| 29 | | 9.9 |
| 30 | | 10.9 |
| 31 | | 3.4 |
| 32 | | 3.2 |
| 33 | | 8.1 |
| 34 | | 8.6 |

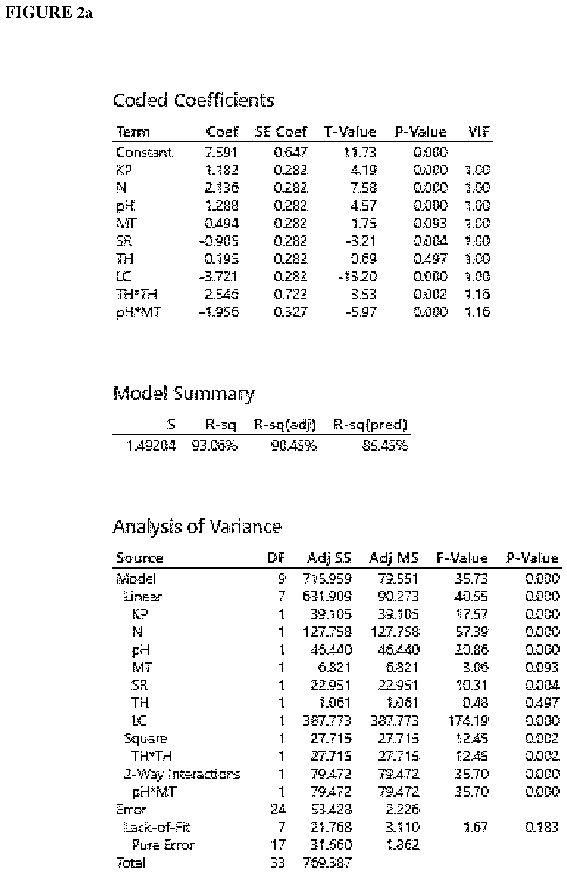

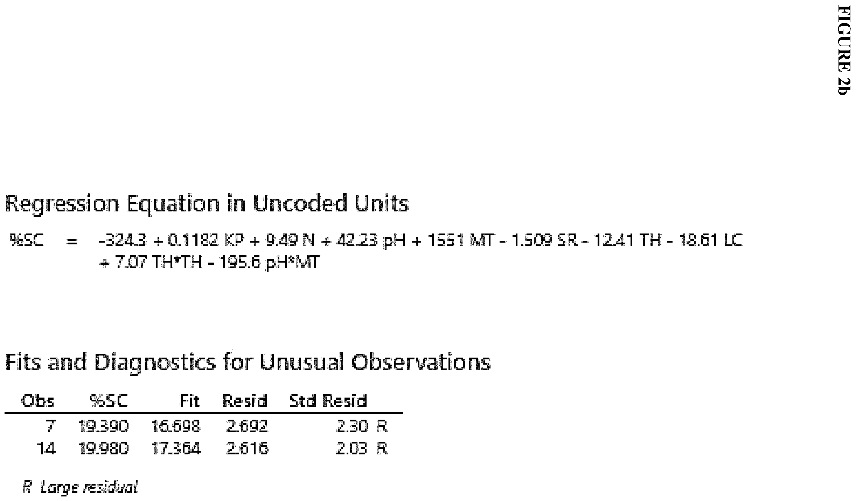

The data from the above results, interpreted through statistical processing, are presented in FIGS. 2a and 2b. FIG. 2a shows the results of experiments conducted with a statistical design to determine the effect of seven factors (KP to LC) on %SC of pDNA. When applying the statistical hypothesis testing criterion of =0.05, it was found that all major factors except MT and TH had a direct impact on %SC, and that interaction and square factors such as pH*MT and TH*TH also influenced %SC. Specifically, it was determined that for KP (potassium phosphate), N (NaCl), and pH, the stability of pDNA increased as the concentration increased within the selected range.

For SR (sucrose) and LC (leucine), it was determined that lower concentrations within the selected range led to increased pDNA stability.

In the case of MT (mannitol), due to its interaction with pH, pDNA stability was found to be highest when the concentration of MT was low and the pH value was high.

According to these findings, a model equation representing the effect of each factor on %SC was derived and presented in FIG. 2b.

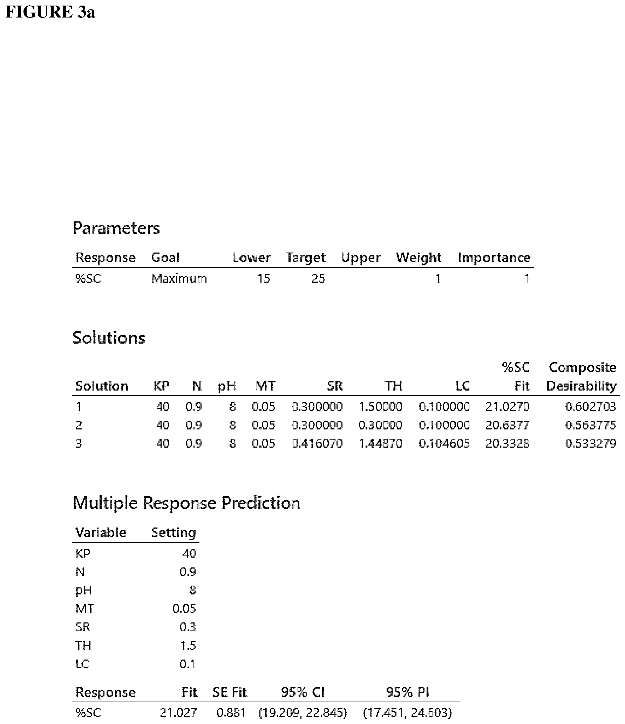

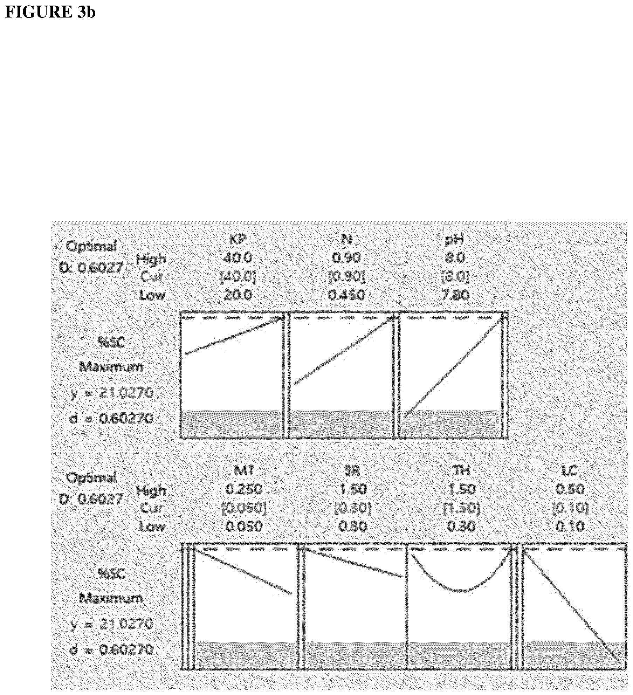

Based on the above analysis, the optimal composition derived is shown in FIGS. 3a and 3b.

### Review

As shown in FIG. 3a, the factors affecting %SC were KP, N, pH, MT, SR, TH, and LC, a total of seven factors. After deriving the most suitable model using these factors, it was confirmed that solution 1 was the optimal model. By applying the model derived from solution 1 and statistically analyzing the equation, it was estimated that the range in which the statistical mean value of %SC would distribute at a 95% confidence level was approximately 19.209 to 22.845. Additionally, it was predicted that the individual values of %SC would range between 17.451 and 26.603 at a 95% confidence level.

Based on the data results, the first composition (solution 1) was selected as the formulation with the highest %SC among the three compositions derived.

Furthermore, the inventors adjusted the concentrations or values of each factor within their respective ranges to maximize the y-value, i.e., %SC (as shown in FIG. 3b).

In the case of sorbitol, the DoE results indicated that its effect on pDNA SC% stability was minimal; therefore, it was fixed at the midpoint concentration of 0.15% in the DoE sorbitol conditions.

| | | |
|---|---|---|
| Formulation C | VM202 DS | 0.5 mg/mL |
| | Potassium phosphate | 40 mM |
| | NaCl | 0.9% |
| | Mannitol | 0.05% |
| | Sucrose | 0.3% |
| | Sorbitol | 0.15% |
| | Trehalose | 1.5% |
| | Leucine | 0.1% |

Example 2-3. Preparation of Liquid Formulation C (Formulation C: potassium phosphate 40 mM, NaCl 0.9%, pH 8.0, mannitol 0.05%, sorbitol 0.15%, sucrose 0.3%, trehalose 1.5%, leucine 0.1%) and Comparison of Formulations A, B, and C (Long-term Stability Test at 70°C)

To verify the stability of Liquid Formulation C under refrigerated conditions (2- 8°C), room temperature storage conditions (20-25°C), and severe conditions (37°C), a long-term stability test was conducted.

The objective was to confirm the long-term stability of pDNA in Liquid Formulations B and C, which were selected through previous screenings, and to evaluate the pDNA stability depending on the production sites of VM202 DS and the combination of Liquid Formulations A, B, and C.

### Test Design

Through the first pre-screening, it was confirmed that mannitol had the most significant pDNA stabilization effect as a single excipient.

Liquid Formulation B, which was prepared by adding only 0.25% mannitol to the existing Liquid Formulation A, and Liquid Formulation C, developed through DoE, were applied to two lots of VM202 DS produced by HX and W companies, and compared with the existing control condition, Liquid Formulation A.

The long-term stability test of Liquid Formulations A, B, and C at 70°C involved sampling on Days 0, 1, 3, and 5 to check SC%.

The test plan for the third long-term stability test is shown in Table 26.

**TABLE 26**

| Third Long-term Stability Test Plan (Liquid Formulations B and C) | | | | | | | | | | | | | | | | | | | |
|---|---|---|---|---|---|---|---|---|---|---|---|---|---|---|---|---|---|---|---|
| Formulati on Code | Temp Conditio ns (°C) | Time Point (month) | | | | | | | | | | | | | | | | | |
| | | T 0 | T0. 5 | T 1 | T1. 5 | T 2 | T2. 5 | T 3 | T4. 5 | T 6 | T1 2 | T1 5 | T1 8 | T2 1 | T2 4 | T2 7 | T3 0 | T3 3 | T3 6 |
| HX1C | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| HX2C | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| W 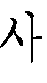 1C | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| W 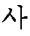 2C | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| HX1B | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| HX2B | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| 1B from Company W | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |
| 2B from Company W | 2-8 | X | - | X | - | X | - | X | - | X | X | X | X | X | X | X | X | X | X |
| | 20-25 | X | X | X | X | X | X | X | X | X | - | - | - | - | - | - | - | - | - |
| | 37 | X | X | X | X | X | X | X | - | - | - | - | - | - | - | - | - | - | - |

| | | | | | | | | | | | | | | | | | | | |
|---|---|---|---|---|---|---|---|---|---|---|---|---|---|---|---|---|---|---|---|
| - "Time point T0.5" refers to 0.5 month (2 weeks). | | | | | | | | | | | | | | | | | | | |

### Results

The SC% (by HPLC) analysis results of the third experiment are shown in Tables 27 and 28 below.

**[Table 27]**

| Comparison of Formulations A, B, and C at 70°C | | | | | | |
|---|---|---|---|---|---|---|
| Formulation Type | DS source | Formulation Code | SC % | | | |
| | | | Day 0 | Day 1 | Day 3 | Day 5 |
| C | HX | HX1C | 97.3 | 82.1 | 46.0 | 25.0 |
| | | HX2C | | | 31.4 | 12.1 |
| | Company W | 1C from Company W | 95.4 | 71.6 | 36.6 | 19.4 |
| | | 2C from Company W | | | 36.6 | |
| | ALD | ALDC | 96.2 | 82.9 | 45.4 | 25.9 |
| B | HX | HX1B | 97.3 | 82.4 | 44.2 | 20.3 |
| | | HX2B | | | 54.4 | 37.0 |
| | Company W | 1B from Company W | | 74.9 | 46.1 | 29.3 |
| | | 2B from Company W | | | | 28.5 |
| | ALD | ALDB | | 70.8 | 22.7 | 6.6 |
| A | HX | HX1A | | 66.6 | 27.4 | 13.1 |
| | | HX2A | | 14.6 | | |
| | Company W | 1A from Company W | | 43.9 | 13.9 | 7.4 |
| | | 2A from Company W | | | | |
| | ALD | ALDA | | 61.8 | 22.7 | 8.9 |

**TABLE 28**

| Round 3 Long-Term Stability Test (Formulation B and C) | | | | | | | | | | | | | | | | | | | |
|---|---|---|---|---|---|---|---|---|---|---|---|---|---|---|---|---|---|---|---|
| Formulat ion Code | Temp Conditi ons (°C) | Time Point (month) | | | | | | | | | | | | | | | | | |
| | | T0 | T0 .5 | T1 | T1 .5 | T2 | T2 .5 | T3 | T4 .5 | T6 | T1 2 | T1 5 | T1 8 | T2 1 | T2 4 | T2 7 | T3 0 | T3 3 | T3 6 |
| HX1C | 2-8 | 97 .6 | | 97 .5 | | 97 .7 | | 97 .1 | | 95 .3 | - | - | - | - | - | - | - | - | - |
| | 20-25 | | 96 .5 | 95 .6 | 95 .0 | 94 .5 | 92 .8 | N/ A | N/ A | N/ A | | | | | | | | | |
| | 37 | | 91 .0 | 86 .9 | 85 .8 | 81 .3 | 77 .7 | 70 .5 | | | | | | | | | | | |
| HX2C | 2-8 | 97 .7 | | 97 .2 | | 96 .3 | | 95 .9 | | 95 .0 | - | - | - | - | - | - | - | - | - |
| | 20-25 | | 94 .4 | 92 .8 | 92 .2 | 90 .9 | 89 .4 | 87 .9 | 84 .4 | 81 .1 | | | | | | | | | |
| | 37 | | 88 .0 | 77 .9 | 78 .1 | 72 .1 | 75 .2 | 59 .8 | | | | | | | | | | | |
| 1C from Company W | 2-8 | 96 .5 | | 96 .8 | | 95 .9 | | 94 .6 | | 93 .4 | - | - | - | - | - | - | - | - | - |
| | 20-25 | | 93 .4 | 9. 5 | 88 .9 | 87 .5 | 87 .2 | 84 .7 | - | - | | | | | | | | | |
| | 37 | | 84 .7 | 76 .6 | 76 .8 | 72 .9 | 70 .2 | 63 .8 | | | | | | | | | | | |
| 2C from Company W | 2-8 | 97 .3 | | 97 .0 | | 96 .5 | | 95 .7 | | 94 .7 | - | - | - | - | - | - | - | - | - |
| | 20-25 | | 94 .6 | 91 .9 | 90 .8 | 89 .4 | 88 .8 | 87 .4 | 81 .9 | 78 .7 | | | | | | | | | |
| | 37 | | 85 .7 | 75 .6 | 77 .3 | 68 .4 | 69 .3 | 60 .0 | | | | | | | | | | | |
| HX1B | 2-8 | 96 .0 | | 96 .8 | | 97 .1 | | 95 .0 | | 94 .5 | - | - | - | - | - | - | - | - | - |
| | 20-25 | | 94 .5 | 92 .8 | 87 .7 | 85 .8 | 84 .3 | 79 .5 | 70 .3 | 61 .3 | | | | | | | | | |
| | 37 | | 83 .7 | 69 .4 | 50 .6 | 47 .6 | 37 .9 | 29 .0 | | | | | | | | | | | |
| HX2B | 2-8 | 96 .0 | | 95 .3 | | 95 .0 | | 94 .7 | | 94 .7 | - | - | - | - | - | - | - | - | - |
| | 20-25 | | 94 .3 | 93 .7 | 93 .0 | 92 .6 | 91 .3 | 89 .6 | 87 .7 | 85 .5 | | | | | | | | | |
| | 37 | | 87 .0 | 85 .5 | 80 .9 | 78 .9 | 83 .0 | 72 .0 | | | | | | | | | | | |
| 1B from Company W | 2-8 | 96 .1 | | 95 .4 | | 95 .7 | | 94 .5 | | 94 .0 | - | - | - | - | - | - | - | - | - |
| | 20-25 | | 94 .2 | 92 .6 | 91 .0 | 89 .7 | 89 .5 | 86 .4 | 83 .2 | 79 .6 | | | | | | | | | |
| | 37 | | 82 .4 | 74 .7 | 69 .2 | 67 .0 | 65 .5 | 61 .1 | | | | | | | | | | | |
| 2B from Company W | 2-8 | 96 .9 | | 96 .9 | | 95 .8 | | 95 .5 | | 92 .0 | - | - | - | - | - | - | - | - | - |
| | 20-25 | | 92 .3 | 90 .1 | 87 .5 | 85 .5 | 85 .2 | 82 .0 | 79 .1 | 76 .3 | | | | | | | | | |
| | 37 | | 82 .5 | 75 .0 | 76 .7 | 68 .4 | 66 .5 | 58 .4 | | | | | | | | | | | |

| | | | | | | | | | | | | | | | | | | | |
|---|---|---|---|---|---|---|---|---|---|---|---|---|---|---|---|---|---|---|---|
| - Only some samples were analyzed for Day 1, 3, and 5, and for the 2-8°C samples. - Samples that could not be analyzed are marked as N/A. | | | | | | | | | | | | | | | | | | | |

As seen in Table 27, Liquid Formulation C showed similar stability across HX, W, and ALD-produced DS at 70°C on Day 5 (average SC 20.6%). In contrast, Liquid Formulation B showed lower SC% stability at Company A (SC 6.6%), while Liquid Formulation A had overall lower SC% stability (9.8%).

This trend was also observed in the long-term stability results shown in Table 28. Liquid Formulation C maintained 60.0-70.5% SC% at 37°C after 12 weeks across all lots of DS from HX and W companies. In contrast, Liquid Formulation B showed significant variation between DS lots, with SC% ranging from 29.0% to 72.0%.

In the long-term stability results at 25°C, Liquid Formulation C showed an average SC% of 79.4% after 6 months, while Liquid Formulation B had an average SC% of 75.7%, indicating that VM202 pDNA was more stable in Liquid Formulation C.

### Review

Based on the above analysis, it appears that Liquid Formulation C, developed through Design of Experiments (DoE), demonstrates less variability across different DS lots compared to Liquid Formulation B and maintains a more stable VM202 pDNA SC% under room temperature conditions (20-25°C).

Additionally, Liquid Formulation C meets the goal of the study, which was to maintain an SC% of 85% or higher after 3 months of storage at 25°C, with an average SC% of 86.7% after 3 months.

Therefore, through the third round of screening, Liquid Formulation C was successfully developed, which can be applied not only to VM202 DS produced by Company A but also to those produced by Companies HX and W.

Considering that all the above experiments were conducted on a small scale, there is a need to verify if the results are reproducible under scale-up conditions.

### Example 2-4: Fourth Round Scale-up Process Adjustment (Scale-up A, C)

To verify whether the liquid formulation C, developed in rounds 1 to 3, can be applied under scale-up conditions and to assess the SC% stability with or without the filter process, the following tests were conducted.

### Test Design

The previous liquid formulation procedure, which was conducted on a 20 mL scale, was scaled up to a 300 mL scale.

To evaluate the impact of the filter process, the 300 mL of BDP produced was divided into two parts: 150 mL underwent filtration, while the other 150 mL was filled into vials without filtration, followed by stress testing.

This process was applied to both liquid formulation C and A to determine the effects of scale-up and the presence or absence of the filter process.

The fourth-round scale-up procedure stress test at 70°C involved sampling on days 0, 1, 3, and 5 to measure SC%.

### Results

The SC% (by HPLC) analysis results of the fourth screening are shown in Table 29.

**TABLE 29**

| Round 4 Scale-Up Process Adjustment | | | | | | | |
|---|---|---|---|---|---|---|---|
| Formulation Type | DS source | Formulation Code | With or Without Filter Process | SC | | | |
| | | | | Day 0 | Day 1 | Day 3 | Day 5 |
| C | HX | HXCW | W | 97.4 | | 35.5 | 13.7 |
| | | HXCWO | WO | | 82.1 | 39.4 | 15.8 |
| A | HX | HXAW | W | 96.5 | | 8.9 | 4.1 |
| | | HXAWO | WO | | | 40.6 | 21.7 |

As shown in Table 29, liquid formulation C maintained a consistent SC% regardless of the filter process. In contrast, for liquid formulation A, the SC% on day 3 was 8.9% when the filter process was applied, compared to 40.6% when the filter process was not used, showing a significant difference of over 30%.

### Review

In actual production processes, the filter process is essential due to bioburden and sterility issues, making it necessary to develop a liquid formulation that can be applied with the filter process.

This test confirmed that liquid formulation C maintains stable SC% even when the filter process is applied. (In contrast, liquid formulation A did not maintain SC% stability when the filter process was applied.)

Therefore, through this example, liquid formulation C was successfully developed as a liquid formulation that can be applied to actual production procedures.

### Conclusion of Example 2

Through four rounds of screening, successful development was made of a VM202 liquid formulation that can be applied to VM202 DS produced by various manufacturers (Rounds 1-2), meets the target SC% stability (Round 3), and is applicable to actual production processes (Round 4).

The long-term stability results of liquid formulation C at 25°C (Round 3) showed an average SC% of 86.7% at 12 weeks, exceeding the target of 85%.

Additionally, this example confirmed that the stability of VM202 pDNA varies depending on the production environment and lot, and that the presence or absence of a filter process also affects pDNA stability. Therefore, when introducing the liquid formulation into actual production, factors such as the production process of VM202 DS, the material of the filters used in production, and the process time should be considered as they may impact the final stability of the liquid DP.

The final liquid formulation C will undergo a long-term stability test (36 months) at 2-8°C if necessary, to further confirm its storage stability. The final formulation will serve as the basis for developing a composition suitable for commercial production.

The composition derived from the four rounds of testing is shown in Table 30 below.

**TABLE 30**

| Final Derived Composition | | | |
|---|---|---|---|
| Development | Ingredient | Concentration | Purpose |
| Formulation B | VM202 DS | 0.5mg/mL | Active Pharmaceutical Ingredient |
| | potassium phosphate | 40mM | Buffering Agent |
| | NaCl | 0.9% | Stabilizer |
| | mannitol | 0.25% | |
| Formulation C | VM202 DS | 0.5mg/mL | Active Pharmaceutical Ingredient |
| | potassium phosphate | 40mM | Buffering Agent |
| | NaCl | 0.9% | Stabilizer |
| | mannitol | 0.05% | |
| | sucrose | 0.3% | |
| | sorbitol | 0.15% | |
| | trehalose | 1.5% | |
| | leucine | 0.1% | |
| To the final pH of 8.0 | | | |

The final liquid formulation C derived from the DoE process (40 mM potassium phosphate, 0.9% NaCl, pH 8.0, mannitol 0.05%, sucrose 0.3%, sorbitol 0.15%, trehalose 1.5%, leucine 0.1%) demonstrated stability that exceeded the DP specification criteria over a 3-month period under storage conditions of 20-25°C (86.7% SC). It is expected that this formulation will remain stable for over 18 months under 2-8°C storage conditions.

The final formulation will be used as a basis for developing a composition that can be applied to commercial production.

### EXAMPLE 3: Development of Liquid Formulation D for Plasmid DNA

Through Examples 1 and 2, it was confirmed that liquid formulations B and C, applied to VM202 DS produced by Companies W and Helixmith, demonstrated a significant level of stability compared to lyophilized formulations.

In this example, a test was conducted to derive the most stable composition for VM202 using the Response Surface Methodology (RSM) with the composition of Liquid Formulation C, and the final liquid formulation derived from RSM was named Liquid Formulation D.

As in previous examples, the stability of VM202 DP was evaluated by the SC% ratio, which is a key factor in determining the quality of VM202.

This study examined the main effects, interaction effects, and curvature effects among the factors in each formulation, with the goal of deriving the optimal composition through further improvements of the composition of Liquid Formulation C.

Liquid Formulation C was previously selected through DoE, where the optimal concentration of each excipient was determined. Based on the interactions identified in Liquid Formulation C, further improvements were made to the composition.

The stability target for this study was to maintain the DP Spec standard (a SC 85%) under storage conditions of 20-25°C for at least 3 months. This target was selected based on research indicating that pDNA SC% degradation accelerates as storage temperature increases. If stability is maintained at 20-25°C for 3 months, it is assumed that the DP will remain stable for over 18 months when stored at 2-8°C.

The improved formulation, Liquid Formulation D, was subjected to a long-term stability test with Liquid Formulation C as the control. Samples were stored at 2-8°C (with sampling every 4 weeks), 20-25°C, and 37°C (with sampling every 2 weeks) for 3 months, with SC% measured throughout the study. (A 3-year plan was set for the 2-8°C storage condition.)

The workflow of the experiment is shown in Table 31 below.

**TABLE 31**

| Workflow | | |
|---|---|---|
| Round No. | Experiment Objective | Purpose |
| 1 | Design of Experiment (RSM) | Derive the final composition by combining factors for pDNA stability (Optimize potassium phosphate, sodium chloride, and leucine (best case)) |
| | | - Derive Formulation D |
| 2 | Long-term Stability Study | Compare the long-term stability of the selected Formulation D with Formulation C under refrigerated, accelerated, and stress conditions. |

### Experiment Overview

The three rounds of screening conducted in this experiment followed the same procedure for Components preparation, BDP Formulation, Vial filling, Sampling, and SC% Analysis as in previous trials.

The vials used in this experiment were prepared as closely as possible to the actual production process. They were washed according to the Vial wash Protocol up to two days before BDP Formulation and stored in a 70°C Dry Oven until use. The rubber stoppers were sterilized by the vendor (West-DAIKYO SEIKO) and used immediately after opening.

### Preparation

The 1X buffer and 2X buffer used in the experiment were prepared no more than three days before the start of each trial's BDP Formulation.

The 2X buffer was prepared with buffer strength, mannitol, sucrose, sorbitol, trehalose, leucine, and arginine concentrations doubled. The NaCl concentration in the 2X buffer was calculated using the following formula based on the Final Formulation's NaCl concentration (A): *(A x 2) - 0.9 = NaCl Conc. of 2X buffer.

The 1X buffer had the same composition as the Final Formulation.

This experiment involved the preparation of a Small Scale buffer (100 mL). Considering the manufacturing tolerances in actual commercial production processes, the 1X and 2X buffers were prepared with a manufacturing tolerance range of ~2%.

The API used in this experiment was VM202 DS produced by Company A, Company C, and Helixmith (lot: 8^{th}, 9^{th}), as well as VM202 DS produced by Company W (W1: 1912063, W2: 1912073). The samples were thawed from a -70°C Deep Freezer to a 2-8°C Refrigerator for 16-18 hours before use.

The thawed DS was mixed with 2X and 1X buffer to prepare BDP with a final pDNA concentration of 0.48-0.52 mg/mL (target 0.5 mg/mL).

The BDP manufacturing process and sampling and analysis procedures are shown in Table 32 below.

**TABLE 32**

| BDP Manufacturing and Sampling Protocols | |
|---|---|
| No. | Procedure |
| 1 | Prepare 2X and 1X buffers according to the Formulation List in Tables 7-12. |
| 2 | Turn on BSC and prepare 2X, 1X, and DS samples - DS is thawed in a 2-8°C refrigerator the day before BDP formulation. |
| 3 | Add 5-7 mL DS to a SPL 50 mL conical tube, record the current volume (A). |
| 4 | Add the same volume of 2X buffer (B) to DS and mix. |
| 5 | Mix on roller mixer for at least 5 minutes, then measure total nucleic acid concentration using nano drop (C). |
| 6 | Calculate the amount of 1X buffer to be added using the formula (((A+B) * (C/500) - A-B)) (D). |
| 7 | Add the calculated amount of 1X buffer in the BSC. |
| 8 | Mix on roller mixer for at least 2 minutes, then measure total nucleic acid concentration 3 times using nano drop (E). |
| 9 | If E is within the range of 0.48-0.52 mg/mL, proceed to step 10. If E > 0.52, recalculate the 1X buffer volume to add using the formula with E instead of C and A+B+D instead of A+B in No. 6, then add the additional 1X buffer and measure the total nucleic acid concentration three times. |
| | If D < 0.48, discard the sample and restart from BDP formulation step 1. |
| 10 | Adjust pH to 8.0 and perform vacuum filtering of BDP |
| | (applied from DOE onwards). |
| 11 | Take 500 µL on Day 0 from the prepared sample, store in a -70°C deep freezer. |
| 12 | Aliquot the remaining sample into 20 mL vials (5 mL each), place in a 70°C oven, and record the time (F). |
| 13 | Sample 200 µL on Days 1, 3, and 5 at the same time as step F. |
| 14 | Store the sampled sample in a -70°C deep freezer. |
| 15 | Thaw the sample in a 2-8°C refrigerator 1 hour before SC% analysis by HPLC. |
| 16 | Record the SC% (by HPLC) analysis results. |

The RSM formulation list used in the first round of screening is shown in Table 33 below.

**TABLE 33**

| First Round Test Design (RSM) | | | | | | | | | |
|---|---|---|---|---|---|---|---|---|---|
| RSM; Random Design | | | | | | | | | |
| No. | K phosphate (mM) | Salt (NaCl) (%) | Leucine | pH | Excipient (%) | | | | API (mg/mL) |
| | | | | | mannitol | sucrose | sorbitol | trehalose | |
| 1 | 37 | 0.85 | 0.095 | 8 | 0.05 | 0.15 | 0.3 | 0.3 | 0.5 |
| 2 | 50 | 0.85 | 0.095 | | | | | | |
| 3 | 37 | 1 | 0.095 | | | | | | |
| 4 | 50 | 1 | 0.095 | | | | | | |
| 5 | 37 | 0.925 | 0.05 | | | | | | |
| 6 | 50 | 0.925 | 0.05 | | | | | | |
| 7 | 37 | 0.925 | 0.14 | | | | | | |
| 8 | 50 | 0.925 | 0.14 | | | | | | |
| 9 | 43.5 | 0.85 | 0.05 | | | | | | |
| 10 | 43.5 | 1 | 0.05 | | | | | | |
| 11 | 43.5 | 0.85 | 0.14 | | | | | | |
| 12 | 43.5 | 1 | 0.14 | | | | | | |
| 13 | 43.5 | 0.925 | 0.095 | | | | | | |
| 14 | 43.5 | 0.925 | 0.095 | | | | | | |
| 15 | 43.5 | 0.925 | 0.095 | | | | | | |

Compositions for liquid formulations C and D used for the second-round long-term stability test are as shown in Table 34, below.

**TABLE 34**

| List of Second Round Long-Term Stability Formulations | | | | | | | | | |
|---|---|---|---|---|---|---|---|---|---|
| Formulations C and D | | | | | | | | | |
| - | K phospha te (mM) | Salt (NaCl ) (%) | p H | Leuci ne (%) | Mannit ol (%) | Sucro se (%) | Sorbit ol (%) | Trehalo se (%) | API (mg/m L) |
| Formulati on C | 40 | 0.9 | 8 | 0.1 | 0.05 | 0.3 | 0.15 | 1.5 | 0.5 |
| Formulati on D | 37 | 1.0 | 8 | 0.05 | 0.05 | 0.3 | 0.15 | 1.5 | 0.5 |

### Analysis

High temperatures accelerate pDNA SC% degradation; therefore, the Liquid DP vials were stored in a 70°C oven, and samples were taken on day 0 and day 5 to analyze SC% by HPLC. The sampled vials were stored immediately in a -70°C Deep Freezer, and the samples were thawed in a 2-8°C cold room 1 hour before the HPLC analysis.

### Long-term Stability Test

To verify long-term stability under various temperature conditions, stability tests were conducted at 2-8°C, 20-25°C, and 37°C. The sampling plan was followed as outlined in Table 36, and the SC% was analyzed.

The 2-8°C samples were stored in the HX cold room, the 25°C samples were stored at room temperature in Lab 530, and the 37°C samples were stored in a 37°C incubator.

To maintain consistent sampling conditions, each vial (filled with 5 mL) was sampled up to 100 µL at a time, with a maximum of 10 samplings before the vial was discarded.

### Test Results and Review

### EXAMPLE 3-1: First Round Test Design (RSM; Response Surface Methodology)

A response surface methodology was designed to secure the optimal composition of factors contributing to pDNA stability using Formulation C, which was developed in previous studies. This was conducted with the objective of identifying the optimal composition and combination by examining the main effects, interactions, and curvature effects among various factors such as excipients, buffering agents, pH, and salts.

### Test Design

The equation for the final composition of Formulation C derived in Examples 1 and 2 was secured, and the degree to which each factor affects stability was identified.

Using the obtained data, the interactions among factors that can be confirmed during RSM design were considered, and the optimization of the final composition was carried out to determine the best possible combination in terms of pDNA stability.

The target factors for the optimal composition identified through DOE are as follows:
Potassium phosphate, Sodium chloride, and Leucine

The SC% (by HPLC) analysis results using the first-round test design (RSM) are shown in Table 35 below.

**TABLE 35**

| Results of the First Round Test Design (RSM) | | | |
|---|---|---|---|
| No. | Formulation code | SC% | |
| | | Day 0 | Day 5 |
| 1 | RSM1 | 95.3 | 15.5 |
| 2 | RSM2 | | 8.7 |
| 3 | RSM3 | 95.4 | 16.6 |
| 4 | RSM4 | | 13.3 |
| 5 | RSM5 | | 18.8 |
| 6 | RSM6 | | 7 |
| 7 | RSM7 | | 16.9 |
| 8 | RSM8 | | 12 |
| 9 | RSM9 | | 7.7 |
| 10 | RSM10 | | 9.8 |
| 11 | RSM11 | | 8.1 |
| 12 | RSM12 | | 8.9 |
| 13 | RSM13 | | 7.8 |
| 14 | RSM14 | | 9.3 |
| 15 | RSM15 | | 9.7 |

### Results

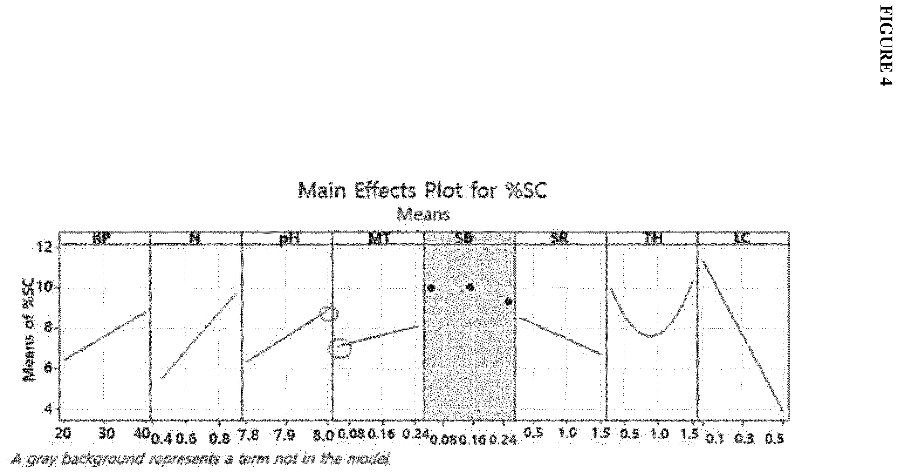

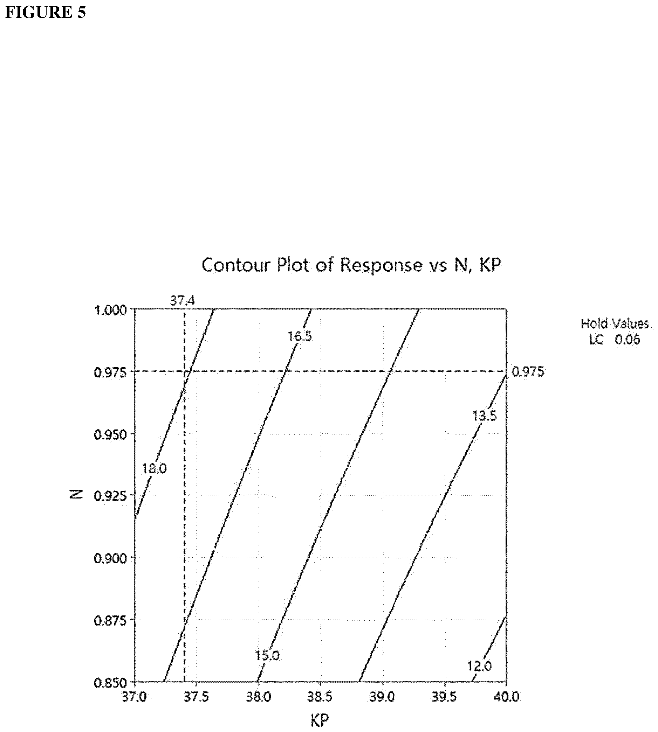

The data from the above results, analyzed through statistical processing, are shown in FIGS. 4 and 5.

### Review

FIG. 4 illustrates the effects of the factors used in pDNA preparation (e.g., KP, NaCl, pH, etc.) on the %SC. As shown in FIG. 4, all factors except for SB had some influence on the %SC. Among them, potassium phosphate (KP), sodium chloride (N), and leucine (LC) were identified as the factors having particularly strong effects, based on visual evaluation. Therefore, these three factors were used in RSM design for formulation optimization, while the other excipients were kept consistent with the optimized composition established through the DOE for Formulation C in previous studies.

The optimal composition of the remaining excipients was selected based on the main effects, interactions, and other relevant factors.

FIG. 5 illustrates the impact of varying concentrations of NaCl and KP on %SC, with leucine fixed at 0.06%. As shown in FIG. 5, the higher the concentration of sodium chloride (NaCl, N) and the lower the concentration of potassium phosphate (KP), the higher the %SC. Specifically, when leucine (LC) is at a concentration of 0.06%, the optimal concentration ranges for potassium phosphate (KP) and sodium chloride (NaCl, N) are 37.0-37.3 mM and 0.975-1.0%, respectively. Therefore, based on these results, the upper limit for leucine was set at 0.055%, and the final concentration was set at 0.05% (leucine range: 0.045~0.055%).

The final composition of Liquid Formulation D was as follows:
Liquid Formulation D: potassium phosphate 37 mM, NaCl 1.0%, pH 8.0, mannitol 0.05%, sorbitol 0.15%, sucrose 0.3%, trehalose 1.5%, leucine 0.05%

### Second Round Long-Term Stability Test (Liquid Formulation D)

### Test Overview

This test was conducted to verify the long-term stability of pDNA in the composition of Liquid Formulation D, selected through the previous DOE, and to compare the long-term stability of pDNA between Liquid Formulation D and Liquid Formulation C.

### Test Design

The composition of Liquid Formulation D was derived by optimizing the concentrations of potassium phosphate, sodium chloride, and leucine from the original Liquid Formulation C. This formulation was then applied as the test composition, with Liquid Formulation C serving as the control for comparison.

Liquid Formulations C and D were stored at 2-8°C with samples taken every 4 weeks, and at 20-25°C and 37°C with samples taken every 2 weeks, for a period of 3 months to measure the SC% (2-8°C storage is planned for 2 years).

The test was conducted using four lots of DS: two lots from HX and two lots from Company W.

The plan for the second-round long-term stability test is provided in Table 36 below.

**TABLE 36**

| Plan for Second-Round Long-Term Stability Test (Formulations D and C) | | | | | | | | | | | | | | | | |
|---|---|---|---|---|---|---|---|---|---|---|---|---|---|---|---|---|
| Formulatio n Code | Temp. Conditio n (°C) | Time Point (month) | | | | | | | | | | | | | | |
| | | T 0 | T0. 5 | T 1 | T1. 5 | T 2 | T2. 5 | T 3 | T4. 5 | T 6 | T 9 | T1 2 | T1 5 | T1 8 | T2 1 | T2 4 |
| HX1D | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | | |
| HX2D | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |
| 1D from Company W | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |
| 2D from Company W | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |
| HX1C | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |
| HX2C | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |
| 1C from Company W | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |
| 2C from Company W | 2-8 | X | - | X | - | X | - | X | - | X | X | X | X | X | X | X |
| | 20-25 | X | X | X | X | X | X | X | X | X | - | - | - | - | - | - |
| | 37 | X | X | X | X | X | X | X | - | - | - | - | - | - | - | - |

| | | | | | | | | | | | | | | | | |
|---|---|---|---|---|---|---|---|---|---|---|---|---|---|---|---|---|
| - Time point T0.5 = 0.5 month (2 weeks). | | | | | | | | | | | | | | | | |

### Test Results

The SC% (by HPLC) analysis results for the second round of tests are shown in Table 37 below.

**TABLE 37**

| Second Round Long-Term Stability Test Results (Formulations and C) D | | | | | | | | | | | |
|---|---|---|---|---|---|---|---|---|---|---|---|
| Formulation Code | Temp. Condition (°C) | Time Point (month) | | | | | | | | | |
| | | T0 | T0.5 | T1 | T1.5 | T2 | T2.5 | T3 | T4.5 | T6 | T9 |
| HX1D | 2-8 | 97.5 | | 97 | | 97.2 | | 96.8 | | 96.7 | |
| | 20-25 | 97.5 | 96.5 | | | | | | | | |
| | 37 | 97.5 | 96.9 | 89.3 | 87.3 | 81. 5 | 79.6 | | | | |
| HX2D | 2-8 | 97.5 | | 96.9 | | 97.2 | | 96.9 | | 96.6 | |
| | 20-25 | 97.5 | 97.1 | 96.7 | 93.7 | 93.1 | 91.3 | 91.1 | | | |
| | 37 | 97.5 | 91.7 | 85.8 | 81.6 | 79.6 | 74.8 | | | | |
| 1D from Company W | 2-8 | 97 | | 96.6 | | 96.9 | | 96.9 | | 97.0 | |
| | 20-25 | 97 | 97 | 96.8 | 96 | 95 | 94.1 | 93.8 | | | |
| | 37 | 97 | 94.7 | 90.1 | 85 | 80.4 | 77.6 | | | | |
| 2D from Company W | 2-8 | 96.5 | | 97.2 | | 97.6 | | 97.2 | | 97.0 | |
| | 20-25 | 96.5 | 96.9 | 97.5 | 95.6 | 94.8 | 94.3 | 94.0 | | | |
| | 37 | 96.5 | 94.5 | 94.1 | 95.1 | 94 | 77.8 | | | | |
| HX1C | 2-8 | 97.5 | | 97.3 | | 97.4 | | 96.8 | | 96.0 | 95.7 |
| | 20-25 | 97.5 | 96.3 | 96.2 | 95 | 94.5 | 94 | | | | |
| | 37 | 97.5 | 92.3 | 87.9 | 85.4 | 82 | 75.9 | 70.5 | | | |
| HX2C | 2-8 | 97.8 | | 97.1 | | 97.0 | | 96.3 | | 95.8 | 94.2 |
| | 20-25 | 97.8 | 95.2 | 94.3 | 93 | 91.2 | 90.3 | 87.9 | 86.4 | 83.3 | |
| | 37 | 97.8 | 89.3 | 80.8 | 78 | 73.9 | 69.8 | 59.8 | | | |
| 1C from Company W | 2-8 | 96.8 | | 96.8 | | 96.3 | | 95.5 | | 93.4 | 92.4 |
| | 20-25 | 96.8 | 95 | 93.3 | 92.4 | 91.1 | 90.4 | 84.7 | | | |
| | 37 | 96.8 | 89.2 | 82.6 | 80.7 | 72.9 | 72.5 | 63.8 | | | |
| 2C from Company W | 2-8 | 97.5 | | 97.0 | | 969. | | 96.3 | | 94.7 | 93.6 |
| | 20-25 | 97.5 | 95.3 | 94.2 | 93 | 92.1 | 91.5 | 87.4 | 85.9 | 84.8 | |
| | 37 | 97.5 | 89.7 | 82.3 | 80.8 | 74.7 | 71.8 | 60 | | | |

| | | | | | | | | | | | |
|---|---|---|---|---|---|---|---|---|---|---|---|
| - Liquid Formulation D (HX1, 25°C) dropped after 4 weeks, and Liquid Formulation C (HX1, 25°C) dropped after 6 weeks. | | | | | | | | | | | |

**TABLE 38**

| Second Round Long-Term Stability Test Results (%SC Average Values) | | | | | | | | | | |
|---|---|---|---|---|---|---|---|---|---|---|
| Formulation Code | Temp. Condition (°C) | Time Point (month) | | | | | | | | |
| | | T0 | T0.5 | T1 | T1. 5 | T2 | T2.5 | T3 | T4.5 | T6 |
| Formulation D | 2-8 | 97.1 | | 96.9 | | 97.2 | | 97.0 | | |
| | 20-25 | 97.1 | 96.8 | 96.5 | 94.8 | 94.3 | 93.2 | 92.8 | 92.5 | 89.8 |
| | 37 | 97.1 | 94.4 | 89.8 | 84.8 | 81.4 | 77.5 | 71.9 | | |
| Formulation C | 2-8 | 97.3 | | 97.1 | | 96.6 | | 96.2 | | 95.0 |
| | 20-25 | 97.3 | 95.5 | 94.5 | 93.3 | 92.2 | 91.4 | 89.4 | 86.4 | 84.1 |
| | 37 | 97.3 | 83.4 | 81.2 | 91.2 | 76.7 | 72.5 | 66.4 | | |

As shown in Table 38, Liquid Formulation C exhibited an average SC stability of 84.1% at 20-25°C after 24 weeks, whereas Liquid Formulation D exhibited an SC stability of 89.8% under the same conditions. Additionally, Liquid Formulation C demonstrated an average SC stability of 66.4% at 37°C after 12 weeks, while Liquid Formulation D showed an SC stability of 71.9% under the same conditions.

### Review

Based on the results above, it was confirmed that Liquid Formulation D, which is an improved version of Liquid Formulation C using RSM, increased SC stability by 5.9% under ambient conditions (20-25°C) and by 5.5% under accelerated conditions (37°C) compared to Formulation C.

Additionally, Liquid Formulation C met the test goal of maintaining SC stability above 85% after 3 months of storage at 25°C, with an SC stability of 89.4%. Liquid Formulation D further improved this stability, achieving 92.8% SC stability after 3 months of storage. (Average % over 3 months)

Therefore, Liquid Formulation D was successfully developed as an optimized formulation that maintains pDNA stability much better than Liquid Formulation C.

### Conclusion of Example 3

Through Example 3, it was confirmed that the final formulation, Liquid Formulation D, derived from a focus on pDNA stability, maintained pDNA stability much more effectively under both accelerated (25°C) and stress (37°C) conditions compared to Liquid Formulation C identified in previous studies.

While Liquid Formulation C achieved an SC stability of 89.4% after 3 months at 25°C, exceeding the VM202 DP spec requirement of 85%, the improved Liquid Formulation D achieved an even higher SC stability of 92.8% under the same conditions, making it a more reliable formulation for maintaining pDNA stability in liquid form.

Given that the pre-improvement Liquid Formulation C was applicable to VM202 DS produced by various manufacturers and met the target SC stability for the test, and was also suitable for actual production processes, it can be concluded that the improved Liquid Formulation D also provides the same effects while offering higher stability.

This indicates that the optimized combination of components in the liquid formulation synergistically enhances pDNA stability, and it is anticipated that it can remain stable for up to 2 years or longer under refrigerated storage conditions for the DP storage temperature.

### Example 4: Long-Term Stability Test of Pre-Filled Syringe Formulation

A long-term stability test was conducted using Liquid Formulations C and D, developed through Examples 1 to 3, to determine the stability when formulated as pre-filled syringes.

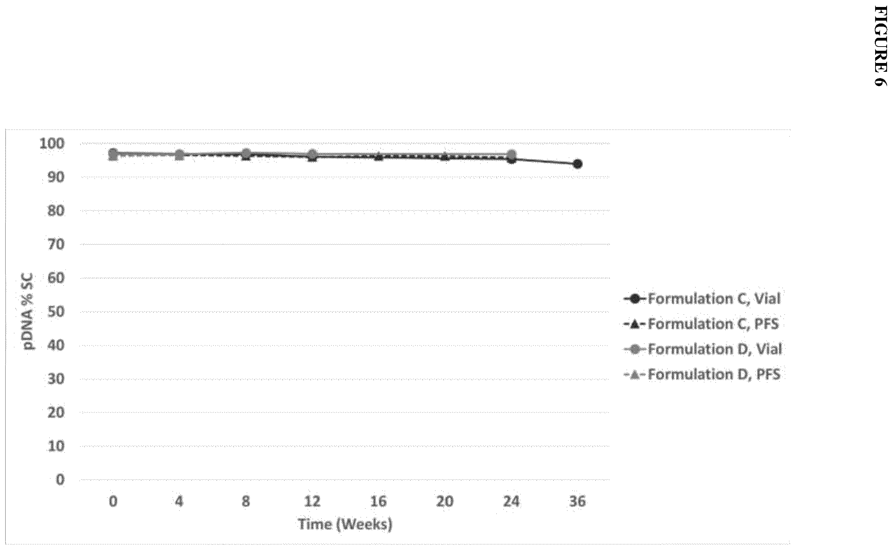

Liquid Formulations C and D, containing 0.5 mg/ml of plasmid DNA, were each formulated into vials and pre-filled syringes, and the samples were stored at 2-8°C, with SC% measured at 4-week intervals for 3 months. The results are shown in Table 39 and FIG. 6.

**TABLE 39**

| **Formulation** | **Presentation** | **Time (Week)** | | | | | | | |
|---|---|---|---|---|---|---|---|---|---|
| | | **0** | **4** | **8** | **12** | **16** | **20** | **24** | **36** |
| C | Vial | 97.6 | 97.5 | 97.7 | 97.1 | | | 95.3 | 95.7 |
| | | 97.7 | 97.2 | 96.3 | 95.9 | | | 95.0 | 94.2 |
| | | 96.5 | 96.8 | 95.9 | 94.6 | | | 93.4 | 92.4 |
| | | 97.3 | 97.0 | 96.5 | 95.7 | | | 94.7 | 93.6 |
| | | 97.3 | 97.0 | 97.1 | 96.4 | | | 96.7 | |
| | | 97.9 | 96.9 | 97.6 | 96.7 | | | 96.5 | |
| | | 97.1 | 96.8 | 96.7 | 96.3 | | | | |
| | | 97.6 | 97.0 | 97.2 | 96.8 | | | | |
| | | 96.6 | 96.3 | 96.3 | 95.6 | 95.3 | 94.6 | 96.2 | |
| | | 96.3 | 96.5 | 96.9 | | | | | |
| | **Average (vial)** | 97.2 | 96.9 | 96.8 | 96.1 | 95.3 | 94.6 | 95.4 | 94.0 |
| | PFS | 96.6 | 96.3 | 96.4 | 96.0 | 96.3 | 96.2 | 95.8 | |
| | | 96.3 | 96.7 | 96.3 | | | | | |
| | **Average (PFS)** | 96.5 | 96.5 | 96.4 | 96.0 | 96.3 | 96.2 | 95.8 | |
| D | Vial | 97.5 | 97.0 | 97.2 | 96.8 | | | 96.7 | |
| | | 97.5 | 96.9 | 97.2 | 96.9 | | | 96.6 | |
| | | 97.0 | 96.6 | 96.9 | 96.9 | | | 97.0 | |
| | | 96.5 | 97.2 | 97.6 | 97.2 | | | 97.0 | |
| | | 96.3 | 96.5 | 96.7 | | | | | |
| | **Average (vial)** | 97.0 | 96.8 | 97.2 | 97.0 | | | 96.8 | |
| | PFS | 96.3 | 96.6 | 96.6 | | | | | |
| | **Average (PFS)** | 96.3 | 96.6 | 96.6 | | | | | |

As shown in Table 39 and FIG. 6, both vials and pre-filled syringes maintained about an SC of 95 % after more than 6 months (24 weeks) at 2-8°C, indicating no difference in storage stability when manufactured as pre-filled syringes.

### Example 5: Stability Test of Formulations C and D with Various Plasmids

To verify whether the liquid formulations C and D, developed in Examples 2 and 3, also provide the same stability for plasmids other than VM202, experiments were conducted. The plasmids used were pCK-SDF-1a (WO2016/048105A1), pTx-IGF-1X10 (WO2020/016655A2), and pCMV3-cMet-flag (Sino Biological Inc., China). The experiments were conducted in the same manner as in the previous examples. SC% was measured after storing the liquid formulations C and D of the three plasmids at 2-8°C and 25°C for 4 weeks.

The results are shown in Table 40.

**TABLE 40**

| pCK-SDF-1a | | | | | |
|---|---|---|---|---|---|
| Formulation | Temp. (°C) | DP lot no. | 0 week | 2 weeks | 4 weeks |
| C | 2-8 | FCV220715-PI01 | 81.4 | 81.7 | 81.4 |
| | 25 | | 81.4 | 80.6 | 79.0 |
| D | 2-8 | FDV220715-PI01 | 79.8 | 79.6 | 79.3 |
| | 25 | | 79.8 | 78.9 | 77.0 |

| pTx-IGF-1X10 | | | | | |
|---|---|---|---|---|---|
| Formulation | Temp. (°C) | DP lot no. | 0 week | 2 weeks | 4 weeks |
| C | 2-8 | FCV220715-PM01 | 98.6 | 99.0 | 99.1 |
| | 25 | | 98.6 | 98.8 | 98.0 |
| D | 2-8 | FDV220715-PM01 | 98.3 | 99.1 | 99.1 |
| | 25 | | 98.3 | 98.7 | 98.0 |

| pCMV3-cMet-flag | | | | | |
|---|---|---|---|---|---|
| Formulation | Temp. (°C) | DP lot no. | 0 week | 2 weeks | 4 weeks |
| C | 2-8 | FCV220715-PC01 | 92.3 | 91.3 | 92.0 |
| | 25 | | 92.3 | 90.6 | 91.1 |
| D | 2-8 | FDV220715-PC01 | 94.6 | 94.0 | 94.5 |
| | 25 | | 94.6 | 92.9 | 93.0 |

As shown in Table 40, all three plasmids exhibited high stability in both liquid formulations C and D. Particularly, for pCK-SDF-1a, even brief exposure to the initial composition with 0.9% NaCl significantly reduced stability (with SC% dropping to 80%). However, in formulations C or D, the SC% remained highly stable over 4 weeks without significant reduction, demonstrating that liquid formulations C or D, especially formulation D, can maintain the stability of even very unstable plasmid DNA.

### EXAMPLE 6: Test for Long-Term Stability of Pre-Filled Syringe Formulations Using Various Plasmids

The long-term stability of pre-filled syringe formulations containing the three plasmids used in Example 5 - pCK-SDF-1a (WO2016/048105A1), pTx-IGF-1X10 (WO2020/016655A2), and pCMV3-cMet-flag (Sino Biological Inc., China) - was evaluated. Each plasmid was formulated in Liquid Formulation D and filled into pre-filled syringes. The syringes were then stored for 4 weeks, and the stability of the plasmids was measured using the percentage of supercoiled plasmid DNA (% SC) as the stability indicator.

The results are summarized in Table 41.

**TABLE 41**

| pCK-SDF-1a | | | | | |
|---|---|---|---|---|---|
| Formulation | Temperature (°C) | DP lot no. | 0 week | 2 weeks | 4 weeks |
| D | 2-8 | FDS220715-PI01 | 79.8 | 79.6 | 79.6 |
| | 25 | | 79.8 | 79.3 | 78.3 |

| pTx-IGF-1×10 | | | | | |
|---|---|---|---|---|---|
| Formulation | Temperature (°C) | DP lot no. | 0 week | 2 weeks | 4 weeks |
| D | 2-8 | FDS220715-PM01 | 98.3 | 98.8 | 99.0 |
| | 25 | | 98.3 | 98.6 | 97.8 |

| pCMV3-cMet-flag | | | | | |
|---|---|---|---|---|---|
| Formulation | Temperature (°C) | DP lot no. | 0 week | 2 weeks | 4 weeks |
| D | 2-8 | FDS220715-PC01 | 94.6 | 93.9 | 94.5 |
| | 25 | | 94.6 | 93.2 | 94.1 |
| | | | | | |

Consistent with the findings in Example 5, the data of Table 41 shows that all three plasmids maintained high stability over the 4-week storage period when formulated in Liquid Formulation D and stored in pre-filled syringes.

## Claims

1. A liquid formulation of plasmid DNA, comprising 30-50 mM potassium phosphate, 0.5-1.5% NaCl, and plasmid DNA, with a pH of 7.5-8.5.

2. The liquid formulation of plasmid DNA according to claim 1, wherein the liquid formulation comprises 40 mM potassium phosphate.

3. The liquid formulation of plasmid DNA according to claim 1, wherein the liquid formulation comprises 0.9% NaCl.

4. The liquid formulation of plasmid DNA according to claim 1, wherein the liquid formulation has a pH of 8.0.

5. The liquid formulation of plasmid DNA according to claim 1, wherein the plasmid DNA in the liquid formulation maintains 80-100 SC% (supercoiled %) after storage at 20-30°C for 6 months.

6. The liquid formulation of plasmid DNA according to claim 1, further comprising sucrose, trehalose, mannitol, sorbitol, leucine, or a combination thereof.

7. The liquid formulation of plasmid DNA according to claim 1, further comprising 1-2% of sucrose, 0.5-2% of trehalose, 0.01-1% of sorbitol, 0.05-0.5% of leucine, or a combination thereof.

8. The liquid formulation of plasmid DNA according to claim 1, wherein the plasmid DNA is present at a concentration of 0.01-5 mg/mL.

9. The liquid formulation of plasmid DNA according to claim 1, further comprising 0.15-1.5% of mannitol.

10. The liquid formulation of plasmid DNA according to claim 9, wherein the plasmid DNA in the liquid formulation maintains 90-100 SC% after storage at 2-8°C for at least 6 months.

11. The liquid formulation of plasmid DNA according to claim 9, wherein the plasmid DNA in the liquid formulation maintains 60-100 SC% after storage at 15-30°C for at least 3, 4, 5, or 6 months.

12. The liquid formulation of plasmid DNA according to claim 1, further comprising 0.001-0.3% of mannitol, 0.05-0.5% of sorbitol, 0.05-0.5% of sucrose, 0.15-3% of trehalose, 0.01-1% of leucine, or a combination thereof.

13. The liquid formulation of plasmid DNA according to claim 12, wherein the plasmid DNA in the liquid formulation maintains 90-100 SC% after storage at 2-8°C for at least 3, 4, 5, 6, 7, 8, or 9 months.

14. The liquid formulation of plasmid DNA according to claim 12, wherein the plasmid DNA in the liquid formulation maintains 80-100 SC% after storage at 20-30°C for at least 3, 4, 5, 6, 7, 8, or 9 months.

15. The liquid formulation of plasmid DNA according to claim 1, further comprising 0.001-0.3% of mannitol, 0.05-0.5% of sorbitol, 0.05-0.5% of sucrose, 0.15-3% of trehalose, 0.045-0.055% of leucine, or a combination thereof.

16. The liquid formulation of plasmid DNA according to claim 15, wherein the liquid formulation comprises 37-37.3 mM potassium phosphate.

17. The liquid formulation of plasmid DNA according to claim 15, wherein the liquid formulation comprises 0.975-1.0% of NaCl.

18. The liquid formulation of plasmid DNA according to claim 15, wherein the plasmid DNA in the liquid formulation maintains 90-100 SC% after storage at 2-8°C for at least 3, 4, 5, 6, 7, 8, or 9 months.

19. The liquid formulation of plasmid DNA according to claim 15, wherein the plasmid DNA in the liquid formulation maintains 80-100 SC% after storage at 20-30°C for at least 3, 4, 5, 6, 7, 8, or 9 months.

20. The liquid formulation of plasmid DNA according to any one of claims 1 to 19, wherein the liquid formulation is prepared in a form of vials, ampules, bottles, or pre-filled syringes.
